# EUROPEAN PATENT APPLICATION

(11) **EP 2 138 725 A2**
(43) Date of publication of application: **30.12.2009**
(21) Application number: 09008297.5
(22) Date of filing: 25.06.2009
(51) Int. Cl.: F04D 25/02, F04D 25/16

(54) **Device for distributing volatile fluids in air**

(30) Priority: 26.06.2008 US 76012 P
(71) Applicant: Momentum Industries, LLC, Fort Wayne IN 46809 (US)
(72) Inventor: Granger, David A., Syracuse Indiana 46567 (US); Gasteiger, Gary, New Haven Indiana 46774 (US)
(74) Representative: RACKETTE Partnerschaft Patentanwälte

(57) **Abstract**

A device for distributing a volatile fluid into a surrounding environment. In one exemplary embodiment, the distribution device includes a dual centrifugal blower having a first blower and a second blower, both of which are driven by a common motor. Additionally, in one exemplary embodiment, substantially identical components may be used to form both the first and second blowers of the dual centrifugal blower, which reduces manufacturing costs. The dual centrifugal blower may be incorporated into air freshener devices having replaceable canisters or cartridges.

## Description

### BACKGROUND

### 1. Field of the Invention.

The present invention relates to a device for distributing volatile fluids in air, such as an air freshening, air cleaning, or insect repelling device for distributing a scented liquid, a disinfectant, an insecticide, or an insect repellant into a surrounding environment, for example. More particularly, the present invention relates to an improved blower assembly and to associated distribution mechanisms for such devices.

### 2. Description of the Related Art.

Devices for distributing volatile fluids in air typically employ a single fan or blower that operates to move air across a volatile liquid contained in a reservoir, such as a scented liquid, for example. As the liquid evaporates into the air, the blower forces the air, which now contains the volatile liquid, out from the device and into the ambient environment. However, over time, an individual experiences olfactory fatigue, which occurs when an individual's tolerance for the volatile liquid increases, such that the individual may no longer notice the beneficial scent provided by the volatile liquid.

What is needed is a device for distributing volatile fluids in air which is an improvement over the foregoing.

### SUMMARY

The present invention provides a device for distributing a volatile fluid into a surrounding environment. In one exemplary embodiment, the distribution device includes a dual centrifugal blower having a first blower and a second blower, both of which are driven by a common motor. Additionally, in one exemplary embodiment, substantially identical components may be used to form both the first and second blowers of the dual centrifugal blower, which reduces manufacturing costs.

In one exemplary embodiment, the dual centrifugal blower may be used in a device, such as an automatic air freshener, for distributing volatile fluids into an ambient environment. For example, the first blower of the dual centrifugal blower may be actuated to blow a first volatile fluid into the ambient environment, while the second blower of the dual centrifugal blower may be actuated to blow a second volatile fluid into the ambient environment. Additionally, the operation of the first and second blowers may be alternated to substantially continuously switch between conveying the first and second volatile fluids into the ambient environment.

Advantageously, by utilizing a dual centrifugal blower, enhanced air flow may be realized for distributing each volatile fluid as compared to current single fan and/or blower systems that are used to distribute volatile fluids into the ambient environment. Additionally, the dual centrifugal blower system is both more efficient and effective than current single fan and/or blower designs. As a result, less power may be needed to operate the dual centrifugal blower. This allows for the blower system to be used over longer periods of time. Specifically, when utilized in applications in which power is supplied to the system by a source of alternating current, the blower's useful life may be extended. Additionally, when utilized in applications in which power is supplied to the system by a source of direct current, such as an alkaline battery, the useful lives of both the blower and the source of direct current may be substantially increased.

In one form thereof, the present invention provides a dual centrifugal blower, including a motor having a housing and a shaft, the motor operable to alternatingly drive rotation of the shaft in a first direction and in an opposite, second direction; a first impeller secured to the shaft in a first position, the first impeller having a first plurality of non-planar blades and disposed in a first housing having a first outlet; and a second impeller secured to the shaft in a second position diametrically opposed to the first position, the second impeller having a second plurality of non-planar blades and disposed in a second housing having a second outlet; wherein, when the motor drives rotation of the shaft in the first direction, the first impeller is operable to generate an air flow having a greater velocity at the first outlet than the second impeller generates at the second outlet, and when the motor drives rotation of the shaft in the second direction, the second impeller is operable to generate an air flow having a greater velocity at the second outlet than the first impeller generates at the first outlet.

In another form thereof, the present invention provides a device for distributing volatile fluids into the ambient environment, the device including a housing having a first housing outlet and a second housing outlet; a first canister containing a first volatile fluid, the first canister disposed externally of the housing and positioned adjacent to the first housing outlet; a second canister containing a second volatile fluid, the second canister disposed externally of the housing and positioned adjacent to the second housing outlet; and a dual centrifugal blower positioned within the housing, the dual centrifugal blower including a motor having a housing and a shaft, the motor operable to alternatingly drive rotation of the shaft in a first direction and in an opposite, second direction; a first impeller secured to the shaft in a first position, the first impeller having a first plurality of non-planar blades and disposed in a first housing having a first outlet; and a second impeller substantially identical to the first impeller, the second impeller secured to the shaft in a second position diametrically opposed to the first position, the second impeller having a second plurality of non-planar blades and disposed in a second housing having a second outlet; wherein, when the motor drives rotation of the shaft in the first direction, the first impeller is operable to generate an air flow having a greater velocity at the first outlet than the second impeller generates at the second outlet, and when the motor drives rotation of the shaft in the second direction, the second impeller is operable to generate an air flow having a greater velocity at the second outlet than the first impeller generates at the first outlet.

In one form thereof, the first volatile fluid and the second volatile fluid are different from each other and are selected from the group consisting of: a scented liquid, a disinfectant, an insecticide, and an insect repellant. In another form thereof, the first canister further includes a canister body and the first volatile fluid is a first fragrance gel, the first fragrance gel disposed internally of the canister body and defining a passageway extending through the first canister. In yet another form thereof, the first and second housing outlets and the first and second outlets of the dual centrifugal blower are disposed at an angle relative to the passageway. In yet another form thereof, the first outlet of the dual centrifugal blower is positioned adjacent to the first housing outlet and the second outlet of the dual centrifugal blower is positioned adjacent to the second housing outlet. In yet another form thereof, a shroud having a shroud body and a surround extending from the shroud body is provided, the shroud body extending at least partially over the housing and the surround extending entirely around a perimeter of the first canister and the second canister. In yet another form thereof, a first canister isolator and a second canister isolator are positioned within the shroud, each of the first and second canister isolators having cylindrical bodies sized for the receipt of the first canister and the second canisters therein, wherein, with the first and second canisters received within the first and second canister isolators, the first and second canister isolators substantially entirely surround a perimeter of the first and second canisters. In yet another form thereof, the housing further includes a plurality of engagement members positioned adjacent the first and second housing outlets, each of the plurality of engagement members cooperating with the housing to define a plurality of gaps between the engagement members and the housing, the plurality of gaps sized to receive a portion of the first and second canisters, whereby the plurality of engagement members respectively retain the first and second canisters adjacent to the first housing outlet and the second housing outlet. In yet another form thereof, the first canister and the second canister further include canister bodies having opposing upper and lower ends, each of the canister bodies having an outwardly extending rim at the lower end thereof, the outwardly extending rims at least partially received within the gaps defined between the engagement members and the housing.

In yet another form thereof, the present invention provides a device for distributing volatile fluids into the ambient environment, the device including a housing having a first housing outlet and a second housing outlet; at least one first cartridge containing a first volatile fluid, the at least one first cartridge disposed within the housing and positioned adjacent to the first housing outlet; at least one second cartridge containing a second volatile fluid, the at least one second cartridge disposed within the housing and positioned adjacent to the second housing outlet; and a dual centrifugal blower positioned within the housing, the dual centrifugal blower including: a motor having a housing and a shaft, the motor operable to alternatingly drive rotation of the shaft in a first direction and in an opposite, second direction; a first impeller secured to the shaft in a first position, the first impeller having a first plurality of non-planar blades and disposed in a first housing having a first outlet; and a second impeller substantially identical to the first impeller, the second impeller secured to the shaft in a second position diametrically opposed to the first position, the second impeller having a second plurality of non-planar blades and disposed in a second housing having a second outlet; wherein, when the motor drives rotation of the shaft in the first direction, the first impeller is operable to generate an air flow having a greater velocity at the first outlet than the second impeller generates at the second outlet, and when the motor drives rotation of the shaft in the second direction, the second impeller is operable to generate an air flow having a greater velocity at the second outlet than the first impeller generates at the first outlet.

In one form thereof, the first volatile fluid and the second volatile fluid are different from each other and are selected from the group consisting of: a scented liquid, a disinfectant, an insecticide, and an insect repellant. In another form thereof, the housing further includes a clamshell housing having a base and a lid pivotably connected to the base, the first housing outlet and the second housing outlet extending through the lid. In yet another form thereof, each of the at least one first cartridge and the at least one second cartridge further include an outer shell having a rim extending outwardly therefrom and a fragrance gel positioned within the outer shell. In yet another form thereof, the housing further includes a plurality of supports positioned adjacent to one another, the supports cooperating to define gaps therebetween, the rims of each of the at least one first cartridge and the at least one second cartridge received within the gaps. In yet another form thereof, the at least one first cartridge includes a pair of first cartridges positioned within the housing in a mutually facing orientation and the at least one second cartridge includes a pair of second cartridges positioned within the housing in a mutually facing orientation. In yet another form thereof, the first pair of cartridges are spaced apart from one another to define a first air channel therebetween and the second pair of cartridges are spaced apart from one another to define a second air channel therebetween, wherein the first blower outlet is position adjacent to the first air channel and the second blower outlet is positioned adjacent to the second air channel.

### BRIEF DESCRIPTION OF THE DRAWINGS

The above-mentioned and other features and advantages of this invention, and the manner of attaining them, will become more apparent and the invention itself will be better understood by reference to the following description of an embodiment of the invention taken in conjunction with the accompanying drawings, wherein:

Figs. 1-3 are perspective views of a dual centrifugal blower according to an exemplary embodiment of the present invention;

Fig. 4 is an exploded, perspective view of the centrifugal blower system of Figs. 1-3;

Fig. 5 is a perspective view of a dual centrifugal blower according to another exemplary embodiment;

Fig. 6 is an exploded, perspective view of the dual centrifugal blower of Fig. 5;

Fig. 7 is another perspective view of the dual centrifugal blower of Fig. 5 depicting opposing housings of the dual centrifugal blower rotated relative to one another;

Fig. 8 is another perspective view of the dual centrifugal blower of Fig. 5;

Fig. 9 is a top, plan view of the dual centrifugal blower of Fig. 8;

Fig. 10 is a left side, elevational view of the dual centrifugal blower of Fig. 8;

Fig. 11 is a right side, elevational view of the dual centrifugal blower of Fig. 8;

Fig. 12 is a cross-sectional view of the dual centrifugal blower of Fig. 8 taken along line 12-12 of Fig. 10;

Fig. 13 is a front, elevational view of the dual centrifugal blower of Fig. 5;

Fig. 14 is a cross-sectional view of the dual centrifugal blower of Fig. 5 taken along line 14-14 of Fig. 13;

Fig. 15 is a cross-sectional view of the dual centrifugal blower of Fig. 5, taken along line 15-15 of Fig. 13;

Fig. 15A is perspective view of a dual centrifugal blower according to another exemplary embodiment;

Fig. 16 is a front, elevational view of an air freshener according to an exemplary embodiment;

Fig. 17 is cross-sectional view of the air freshener of Fig. 16 taken along line 17-17 of Fig. 16;

Fig. 18 is an exploded, perspective view of the air freshener of Fig. 16 further depicting an additional set of fragrance canisters;

Fig. 19 is a perspective view of the base and housing of the air freshener of Fig. 16;

Fig. 20 is front, elevational view of the components of an air freshener shown in Fig. 19;

Fig. 21 is a top, plan view of the air freshener components shown in Fig. 19;

Fig. 22 is a right side, elevational view of the air freshener components of Fig. 19;

Fig. 23 is a perspective view of the base of the air freshener of Fig. 16;

Fig. 24 is another perspective view of the air freshener base shown in Fig. 23;

Fig. 25 is a front, elevational view of the air freshener base of Fig. 23;

Fig. 26 is a top, plan view of the air freshener base of Fig. 23;

Fig. 27 is a right side, elevational view of the air freshener base of Fig. 23;

Fig. 28 is an elevational view of a pair of fragrance canisters stacked one atop another;

Fig. 29 is another elevational view of the fragrance canisters of Fig. 28;

Fig. 30 is a perspective view of the fragrance canisters of Fig. 28;

Fig. 31 is a cross-sectional view of the fragrance canisters of Fig. 28 taken along line 31-31 of Fig. 28;

Fig. 32 is a cross-sectional view of the fragrance canisters of Fig. 29, taken along line 32-32 of Fig. 29;

Fig. 33 is bottom view of the fragrance canisters of Fig. 28;

Fig. 34 is a top, plan view of the fragrance canisters of Fig. 28;

Fig. 35 is a perspective view of another exemplary embodiment of an air freshener depicting the housing of the air freshener in an open position;

Fig. 36 is a front, elevational view of the air freshener of Fig. 35;

Fig. 37 is a cross-sectional view of the air freshener of Fig. 36 taken along line 37-37 of Fig. 36;

Fig. 38 is another front, elevational view of the air freshener of Fig. 35; and

Fig. 39 is a cross-sectional view of the air freshener of Fig. 38 taken along line 39-39 of Fig. 38.

Corresponding reference characters indicate corresponding parts throughout the several views. The exemplifications set out herein illustrate preferred embodiments of the invention and such exemplifications are not to be construed as limiting the scope of the invention in any manner.

### DETAILED DESCRIPTION

As indicated above, the present invention relates to dual centrifugal blowers that may be used in conjunction with devices, such as air fresheners, for distributing one or more volatile fluids into a surrounding or ambient environment. Set forth below are detailed descriptions of various dual centrifugal blower designs and devices configured for use with the dual centrifugal blowers of the present invention.

### DUAL CENTRIFUGAL BLOWER DESIGN

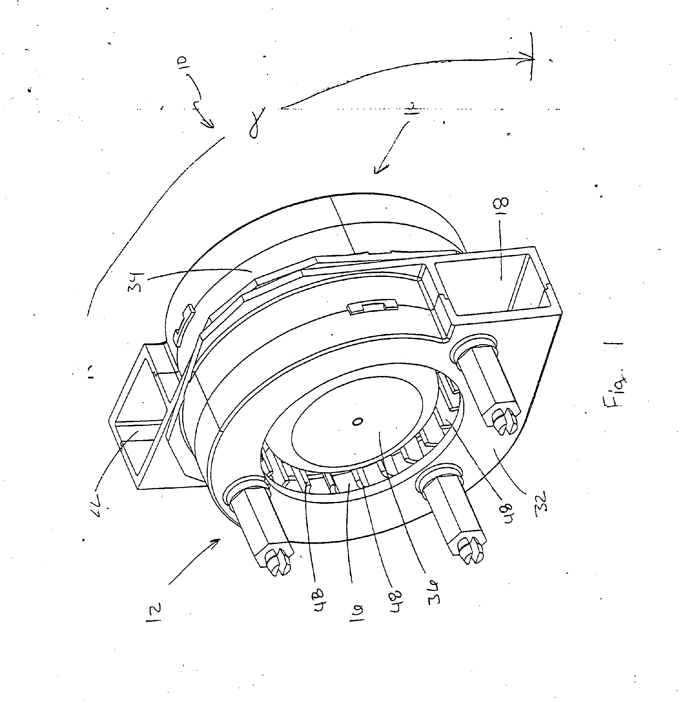

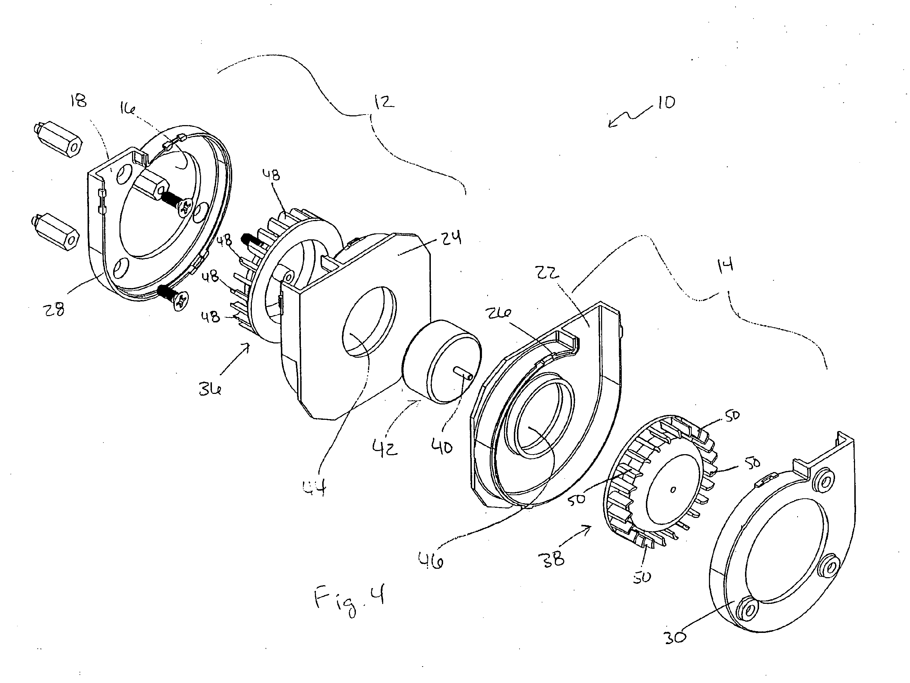

Referring to Figs. 1-3, dual centrifugal blower 10 is shown including opposing first and second blowers 12, 14. As shown in Fig. 4, both of blowers 12, 14 are formed using substantially identical components. Specifically, to form dual centrifugal blower 10, blowers 12, 14 are oriented in a position in which blowers 12, 14 are rotated 180 degrees relative to one another as shown and are secured together as described in detail below. By forming each of blowers 12, 14 using substantially identical components, the overall cost of manufacturing dual centrifugal blower 10 is substantially lessened.

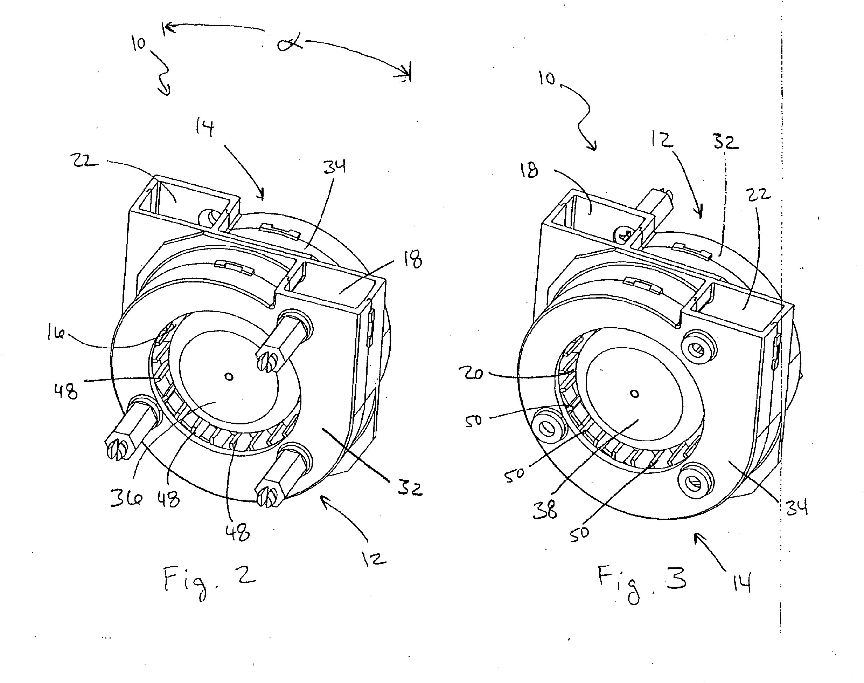

Referring to Figs. 1 and 2, first blower 12 operates by drawing air through inlet 16 and discharging the same through outlet 18. Similarly, referring to Fig. 3, second blower 14 operates by drawing air through inlet 20 and discharging the same through outlet 22. Referring to Fig. 4, outlets 18, 22 of blowers 12, 14, respectively, are formed by the cooperation of inner housing portions 24, 26 and outer housing portions 28, 30. Additionally, inner housing portion 24 and outer housing portion 28 cooperate to define volute 32 of first blower 12. Similarly, inner housing 26 and outer housing 30 cooperate to define volute 34 of second blower 14. Positioned within volutes 32, 34 are fans or impellers 36, 38, respectively.

Impellers 36, 38 are mounted on and rotationally fixed to opposing ends of shaft 40 of motor 42. In one exemplary embodiment, impellers 36, 38 are rotationally fixed to shaft 40 by a splined engagement with shaft 40. As a result of rotationally fixing impellers 36, 38 to shaft 40, both of impellers 36, 38 rotate in the same direction as shaft 40 during operation of motor 42. Further, since impellers 36, 38 of blowers 12, 14 are substantially identical to one another, the curvature of impeller blades 48, 50 is substantially identical. Thus, when impellers 36, 38 are oriented 180 degrees with respect to one another, as shown in Fig. 4, and attached to shaft 40, blades 48, 50 are positioned with the curvature of blades 48, 50 being opposite from one another. As a result, when both impellers 36, 38 are rotated in the same direction, such as by operation of motor 42, only one of impellers 36, 38 creates sufficient airflow to disperse a volatile fluid into the ambient environment.

For example, in one exemplary embodiment, when motor 42 is operated to rotate shaft 40 in a clockwise direction, impeller 36 draws a substantial amount of air through inlet 16, into volute 32, and discharges the air through outlet 18. This airflow generated by blower 12 may then be used to disperse a volatile fluid into the ambient environment. At the same time, impeller 38 is also rotating, but, due to the reversed curvature of blades 50, substantially minimal air flow is generated by blower 14 and it is insufficient to disperse a volatile fluid into the ambient environment. In contrast, if motor 42 is operated to rotate shaft 40 in a counterclockwise direction, impeller 38 draws a substantial amount of air through inlet 20, into volute 34, and discharges the air through outlet 22. This airflow generated by blower 14 may then be used to disperse a volatile fluid into the ambient environment. At the same time, impeller 36 is also rotating, but, due to the reversed curvature of blades 48, substantially minimal air flow is generated by blower 12 and it is insufficient to disperse a volatile fluid into the ambient environment.

In one exemplary embodiment, a friction or press fit is used to connect each blower 12, 14 to motor 42 to form dual centrifugal blower 10. Specifically, referring to Fig. 4, motor 42 is secured to blowers 12, 14 by a friction or press fit between the outer housing of motor 42 and the walls of inner housings 24, 26 defining motor mounting apertures 44, 46, respectively. The friction or press fit between the outer housing of motor 42 and the walls defining motor mounting apertures 44, 46 secures blowers 12, 14 together and forms dual centrifugal blower 10.

Advantageously, by utilizing this connection, the positions of blowers 12, 14 are substantially infinitely variable relative to one another. For example, referring to Figs. 1 and 2, angle α between outlets 18, 22 of blowers 12, 14 may be adjusted to position outlets 18, 22 at different locations by rotating one or both of blowers 12, 14 relative to motor 42 and, correspondingly, relative to one another. This allows for blowers 12, 14 to be used in a variety of different applications where the desired airflow directions generated by blowers 12, 14 varies. While the connection between blowers 12, 14 is described herein with specific reference to a friction fit with motor 42, any other known connection mechanism may be used to connect blowers 12, 14 together to form dual centrifugal blower 10. For example, blowers 12, 14 may be connected together using fasteners, such as screws or bolts, bayonet connections, a snap-fit, a friction-fit, and/or a detent mechanism.

### ALTERNATIVE DUAL CENTRIFUGAL BLOWER DESIGN

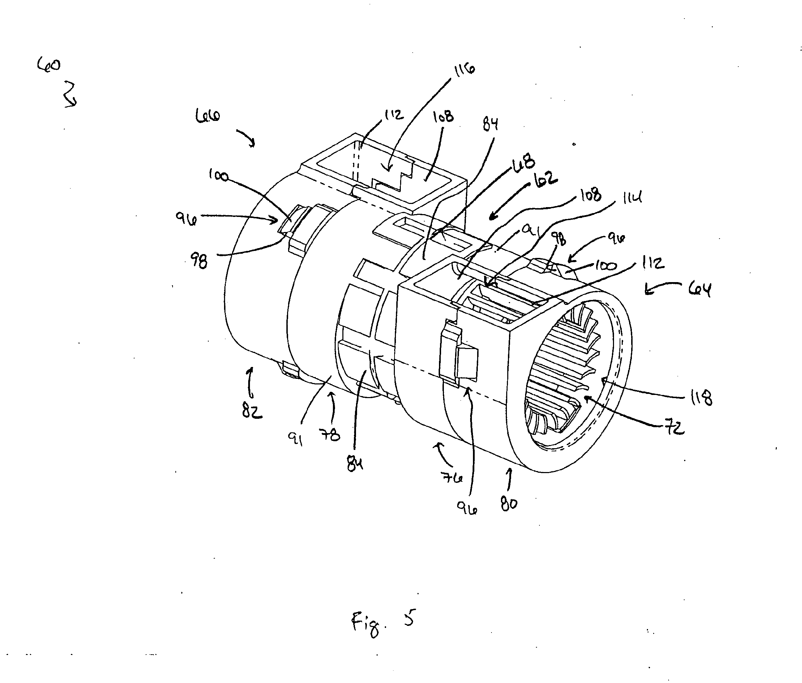

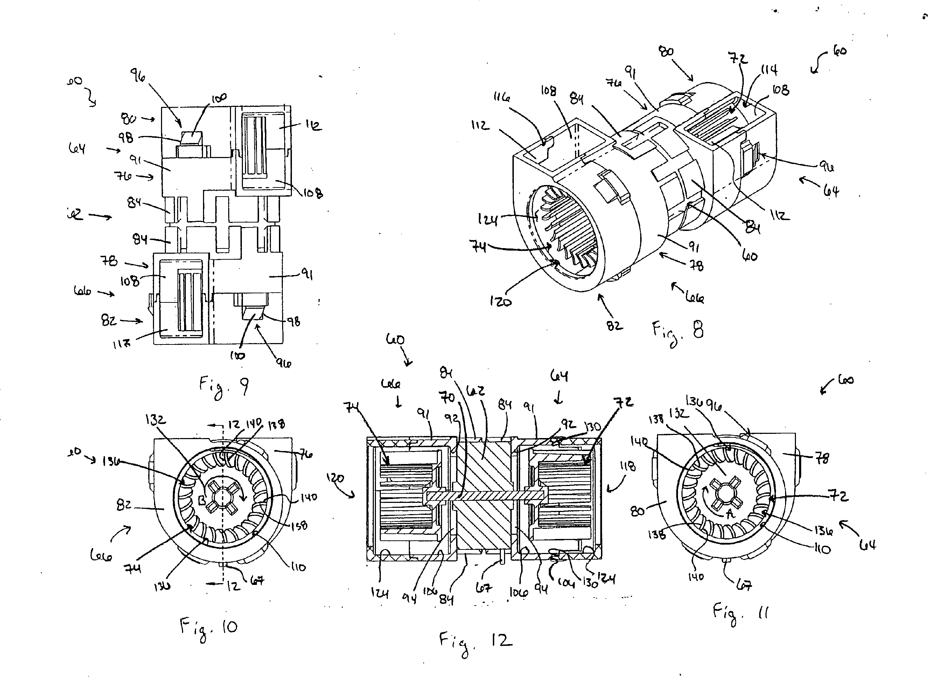

Referring to Figs. 5-15, another exemplary dual centrifugal blower design is shown as dual centrifugal blower 60. Dual centrifugal blower 60 includes several features which are identical or substantially identical to dual centrifugal blower 10 described above. However, for clarity, different reference numerals have been used to identify the components of dual centrifugal blower 60. As shown in Fig. 5, dual centrifugal blower 60 includes motor 62 having blowers 64, 66 connected thereto. In a similar manner as blowers 12, 14 of dual centrifugal blower 10 described in detail above, blowers 64, 66 are formed from substantially identical components and are positioned on motor 62 according to a relative orientation by which first blower 64 and second blower 66 are rotated 180 degrees with respect to one another, as described in detail below.

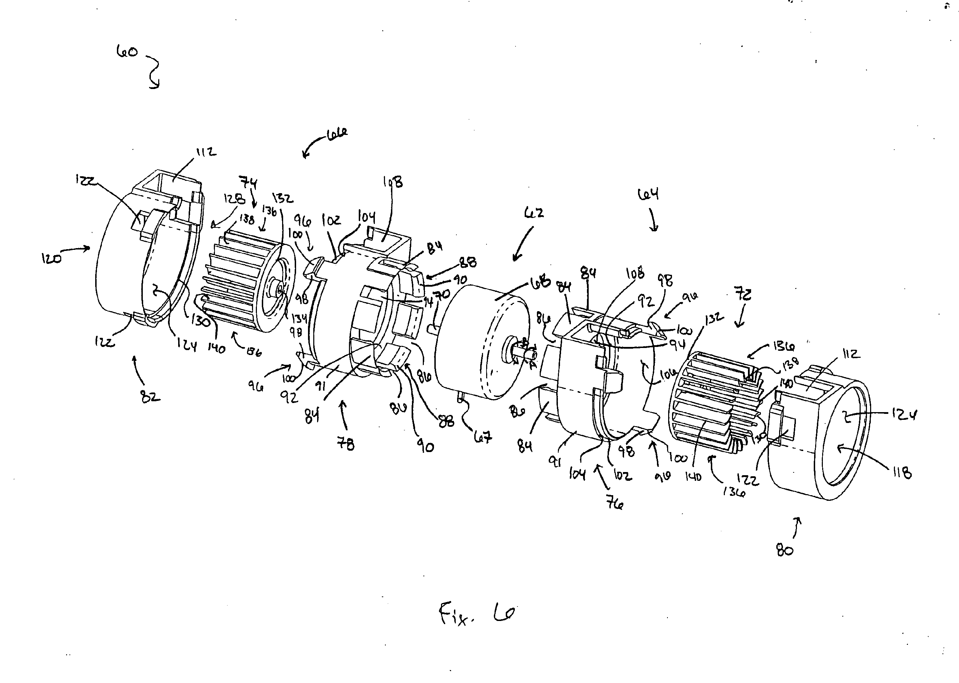

Referring to Fig. 6, motor 62 is an electric motor, which, in exemplary embodiments, may be operated by either direct current or alternating current. Additionally, electric current may be delivered to motor 62 via electrical leads 67. Motor 62 may include a stator and a rotor (not shown) positioned within housing 68. Shaft 70 extends through the rotor of motor 62 and outwardly from opposing sides of housing 68. In one exemplary embodiment, shaft 70 is formed as a single, integral component. In other embodiments, shaft 70 is formed from multiple components. For example, shaft 70 may include a first component or sub-shaft that is rotationally secured to the rotor of motor 62 and that extends from a first side of housing 68. Shaft 70 may also include a second component or sub-shaft that is rotationally secured to the rotor of motor 62 and that extends from a second, opposing side of housing 68. Shaft 70 of motor 62 is designed to receive fans or impellers 72, 74 of blowers 64, 66 thereon, as described in detail below, and functions to drive impellers 72, 74 during operation of dual centrifugal blower 60.

As indicated above, blowers 64, 66 are connected to motor 62. Specifically, referring to Fig. 6, in one exemplary embodiment, blowers 64, 66 include inner housing portions 76, 78 and outer housing portions 80, 82. As used herein, the term "housing" includes any structure that at least partially surrounds an impeller and functions to direct the flow of air generated by the impeller. Inner housing portions 76, 78 of blowers 64, 66 cooperate with outer housing portions 80, 82 to define volutes, as described in detail below. Extending from inner housing portions 76, 78 are a plurality of tabs or projections 84. Projections 84 are configured to be resiliently deformable and are spaced from one another by openings 86. Terminal ends 88 of projections 84 include chamfered surfaces 90, which facilitate the securement of inner housing portions 76, 78 and blowers 64, 66 to motor 62, such as by a friction fit or press fit. Additionally, extending radially inwardly from outer surfaces 91 of inner housing portions 76, 78 and below projections 84 are shoulders 92. Shoulders 92 define shaft openings 94 configured for the receipt of shaft 70 of motor 62 therethrough.

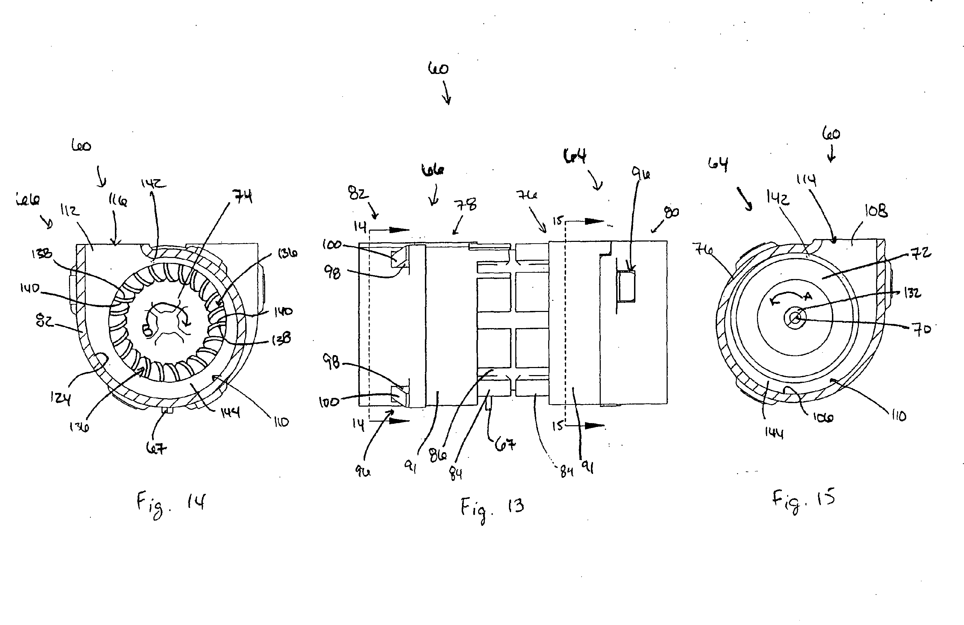

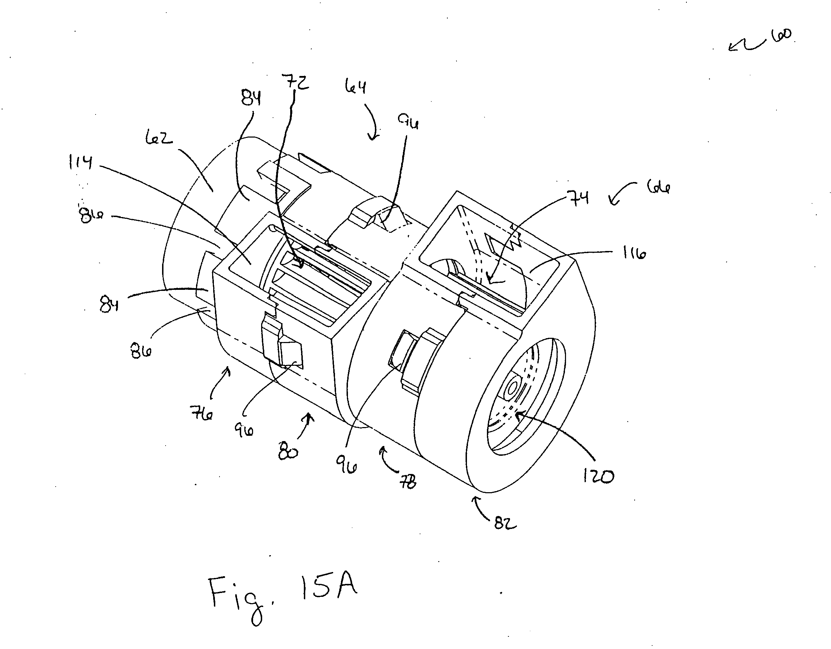

Tabs 96 extend from inner housing portions 76, 78 at the sides of inner housing portions 76, 78 that are opposite projections 84. Tabs 96 are resiliently deformable and include head portions 98 having outer chamfered surfaces 100. Tabs 96 function to secure inner housing portions 76, 78 to outer housing portions 80, 82, as described in detail below. Recesses 102 are formed in outer surfaces 91 of inner housing portions 76, 78 and extend between tabs 96. Recesses 102 are partially defined by ledges 104. Ledges 104 facilitate the securement and alignment of outer housing portions 80, 82 with inner housing portions 76, 78, as described in detail below. Inner housing portions 76, 78 also include inner surfaces 106 and cutouts 108. Inner surfaces 106 of inner housing portions 76, 78 cooperate with inner surfaces 124 of outer housing portions 80, 82 to define volutes 110, shown in Figs. 14 and 15. Additionally, cutouts 108 of inner housing portions 76, 78 cooperate with corresponding cutouts 112 in outer housing portions 80, 82 to define outlets 114, 116, respectively.

Outer housing portions 80, 82 define inlets or inlet openings 118, 120, respectively, formed in one end thereof. Outer housing portions 80, 82 also include openings 122 sized to receive head portions 98 of tabs 96 of inner housing portions 76, 78 therein to secure outer housing portions 80, 82 and inner housing portions 76, 78 together. Outer housing portions 80, 82 also include inner surfaces 124 and cutouts 112. As indicated above, inner surfaces 124 of outer housing portions 80, 82 cooperate with inner surfaces 106 of inner housing portions 76, 78 to define volutes 110. Additionally, cutouts 112 of outer housing portions 80, 82 cooperate with cutouts 108 of inner housing portions 76, 78 to define outlets 114, 116, respectively. Outer housing portions 80, 82 also include recesses 126 extending adjacent to opening ends 128 and opposite inlets 118, 120 of outer housing portions 80, 82. Recesses 126 are formed in inner surfaces 124 and are defined in part by ledges 130. Recesses 126 and ledges 130 of outer housing portion 80, 82 cooperate with recesses 102 and ledges 104 of inner housing portions 76, 78 to facilitate the alignment and centering of outer housing portions 80, 82 and inner housing portions 76, 78 relative to one another.

Impellers 72, 74 are positioned within inner housing portions 76, 78 and outer housing portions 80, 82, respectively. Impellers 72, 74 include hubs 132 having openings 134 formed therein. Openings 134 are sized for receipt of drive shaft 70 of motor 62 therein. In one exemplary embodiment, openings 134 are sized to form a friction-fit with shaft 70 of motor 62. Alternatively, the portions of hubs 132 defining openings 134 may be formed to create a splined engagement with shaft 70 or may otherwise by fixedly connected to shaft 70 in any known manner. Hubs 132 are formed as substantially circular discs and include a plurality of blades 136 extending outwardly therefrom. Specifically, blades 136 are positioned along the outer radial edge of hubs 132 and extend circumferentially around hubs 132. Stated another way, blades 136 are positioned at the outer edges of hubs 132 and extend outwardly therefrom in a direction substantially parallel with shaft 70 and motor 62 when impellers 72, 74 are positioned thereon.

Each of blades 136 of impellers 72, 74 have an arcuate curvature defining concave air moving surfaces 138 and opposing convex surfaces 140. By rotating impellers 72, 74 in the direction of concave air moving surfaces 138, i.e., by rotating impellers in the direction in which concave air moving surfaces 138 are the first to contact air within housings 76, 78, 80, 82, the efficiency of impellers 72, 74 are substantially improved. For example, by utilizing concave air moving surfaces 138 and rotating impellers 72, 74 such that concave air moving surfaces 138 define the leading surfaces of impellers 72, 74 as impellers 72, 74 are rotated, impellers 72, 74 are substantially more efficient, i.e., are capable of moving a greater volume of air per minute, than impellers having blades with substantially planar or flat surfaces or, alternatively, than if impellers 72, 74 are rotated such that convex arcuate surfaces 140 define the leading surface of impellers 72, 74 as impellers 72, 74 move through the air. Thus, when impellers 72, 74 are rotated in a direction such that convex surfaces 138 of blades 136 are the first to contact air within volutes 110, and therefore define the leading surfaces of impellers 72, 74, the efficiency of impellers 72, 74 is substantially reduced, as discussed in detail below.

Referring to Figs. 5, 7, 8, and 13, dual centrifugal blower 60 is shown in its assembled condition. In order to assemble dual centrifugal blower 60, blowers 64, 66 are secured to motor 62. Specifically, inner housing portions 76, 78 of blowers 64, 66 are aligned with and advanced axially along longitudinal axis LA of motor 62 defined by shaft 70. As inner housing portions 76, 78 are advanced in the direction of motor 62, chamfered surfaces 90 of projections 84 contact housing 68 of motor 62. As inner housing portions 76, 78 continue to be advanced in the direction of motor 62, chamfered surfaces 90 facilitate axial alignment of inner housing portions 76, 78 with longitudinal axis LA of motor 62 and projections 84 begin to resiliently expand until projections 84 are positioned substantially entirely on housing 68 of motor 62. Additionally, one of inner housing portions 76, 78 may be aligned with electrical leads 67 such that electrical leads 67 are received within one of openings 86 defined between projections 84. In one exemplary embodiment, inner housing portions 76, 78 may be advanced in the direction of motor 62 such that shaft 70 is received through openings 94 and shoulders 92 of inner housing portions 76, 78 contact housing 68 of motor 62. Once in this position, inner housing portions 76, 78 are fully seated upon motor 62 and frictional securement of motor 62 to inner housing portions 76, 78 is provided.

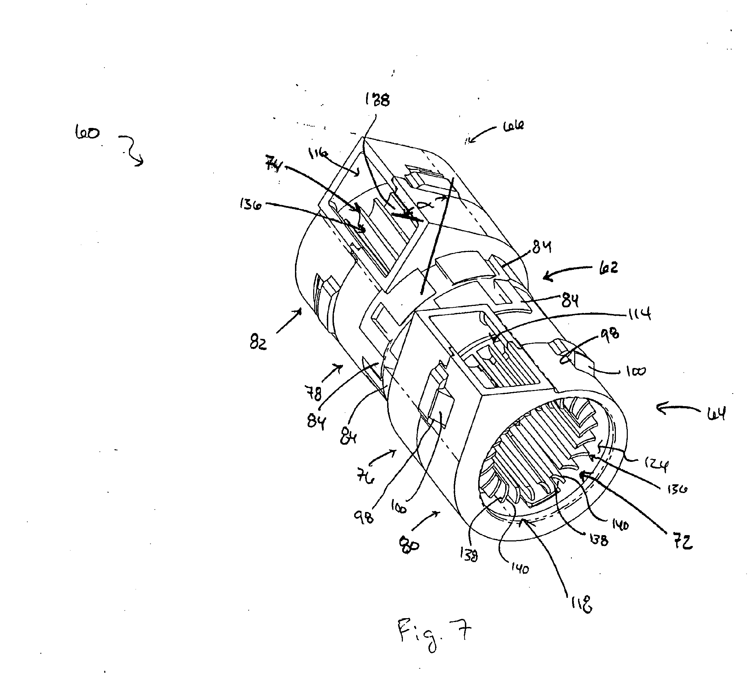

Advantageously, by utilizing a friction-fit connection between inner housing portions 76, 78 and motor 62, the relative rotational positions of blowers 64, 66 are substantially infinitely variable relative to one another, as discussed in detail above with respect to dual centrifugal blower 10. For example, referring to Fig. 7, angle α defined between outlets 114, 116 of blowers 64, 66 may be adjusted to position outlets 114, 116 at different locations by rotating one or both of blowers 114, 116 relative to motor 62 and, correspondingly, relative to one another. This allows for blowers 64, 66 to be used in a variety of different applications where the desired airflow directions generated by blowers 64, 66 varies. While the connection between blowers 64, 66 and motor 62 is described herein with specific reference to a friction fit connection between inner housing portions 76, 78 and motor 62, any other known connection may be used to connect blowers 64, 66 together to form dual centrifugal blower 60. For example, blowers 64, 66 may be connected together using fasteners, such as screws or bolts, a bayonet connection, a snap-fit, a friction-fit, and/or a detent mechanism.

With inner housing portion 76, 78 seated on motor 62, impellers 72, 74 may be secured to shaft 70 of motor 62. Specifically, openings 134 in hubs 132 of impellers 72, 74 are aligned with opposite ends of shaft 70 of motor 62 and impellers 72, 74 are advanced in the direction of motor 62. In this manner, impellers 72, 74 are positioned on shaft 70 such that impellers 72, 74 are diametrically opposed to one another. In one exemplary embodiment, openings 134 of impellers 72, 74 are sized such that impellers 72, 74 are frictionally fit to shaft 70, as indicated above. Alternatively, in other exemplary embodiments, shaft 70 may extend through openings 134 and impellers 72, 74 may be secured to shaft 70 using a suitable fastener, such as a nut or cotter pin. In one exemplary embodiment, opening 134 extending into hubs 132 of impellers 72, 74 have a closed end blind cavity, such that, as impellers 72, 74 are advanced in the direction of motor 62, shaft 70 contacts the portion of hubs 132 defining closed ends 134 of impellers 72, 74 and indicates that impellers 72, 74 are fully seated on shaft 70.

Now, outer housing portion 80, 82 may be secured to inner housing portions 76, 78 to capture impellers 72, 74 therebetween. Specifically, outer housing portions 80, 82 are aligned with longitudinal axis LA of motor 62 and are advanced in the direction of motor 62. As outer housing portions 80, 82 are axially advanced in the direction of inner housing portions 76, 78, head portions 98 of tabs 96 of inner housing portions 76, 78 contact outer housing portions 80, 82. Upon contact with outer housing portions 80, 82, chamfered surfaces of head portions 98 of tabs 96 are advanced along inner surfaces 124 of outer housing portions 80, 82 and begin to cause resilient deformation of tabs 96. As outer housing portions 80, 82 continue to be advanced in the direction of inner housing portions 76, 78, tabs 96 continue to be resiliently deformed until tabs 96 are positioned substantially entirely within openings 122, at which time tabs 96 will return to their original position and head portions 98 of tabs 96 extend through openings 122 in outer housing portions 80, 82. In this manner, tabs 96 function to secure inner housing portions 76, 78 to outer housing portions 80, 82.

Additionally, with inner and outer housing portions 76, 78, 80, 82 in this position, ledges 104, 130 of inner housing portions 76, 78 and outer housing portions 80, 82, respectively, are positioned substantially adjacent one another and the walls of outer housing portions 80, 82 defining recesses 126 are received within recesses 102 of inner housing portions 76, 78. Similarly, the walls of inner housing portions 76, 78 defining recesses 102 are correspondingly received within recesses 126 of outer housing portions 80, 82. Ledges 104, 130 cooperate with the walls defining recesses 102, 126 in inner housing portions 76, 78 and outer housing portions 80, 82, respectively, to help retain inner and outer housing portions 76, 78, 80, 82 in their desired relative positions with respect to one another by limiting movement of inner housing portions 76, 78 relative to outer housing portions 80, 82.

While the assembly of dual centrifugal blower 60 has been described herein with reference to a specific ordering of steps, the components of dual centrifugal blower 60 may be assembled in any order and nothing set forth herein is intended to limit the assembly of dual centrifugal blower 60 to a specific method. For example, inner housing portions 76, 78 may be secured to outer housing portions 80, 82 before inner housing portions 76, 78 are secured to motor 62.

Alternatively, in another exemplary, shaft 70 of motor 62 may extend from a single side of motor 62, as indicated above. In this embodiment, shown in Fig. 15A, blower 64 is secured to motor 62 in the same manner as described in detail above. Blower 66 may then be rotated 180 degrees relative to blower 64 and secured to shaft 70 in a substantially similar manner as described in detail above. Blower 66 may also be modified to eliminate projections 84, as blower 66 no longer connects directly to motor 62. Projections 84 may be replaced by a connection mechanism (not shown) that provides a connection between blower 64 and blower 66. In one exemplary embodiment, the connection mechanism that connects blower 64 and blower 66 forms a rotatable connection therebetween. Further, blower 64 may include a separate inlet (not shown), such as inlet 118, and blower 66 may be spaced apart from blower 64 to allow for air to enter the inlet. Alternatively, blower 64 may be spaced slightly apart from motor 62 such that gaps 86 formed between projections 84 allow for air to pass therethrough and enter blower 64.

Referring to Figs. 1-15, in operation, motor 62 of dual centrifugal blower 60 can be activated to selectively operate blowers 64, 66. Specifically, as a result of securing substantially identical blowers having impellers with arcuate blades in positions that are 180 degrees rotated from one another, i.e., in positions that are diametrically opposed, impellers 72, 74 are positioned such that concave air moving surfaces 138 of blades 136 of impeller 72 of blower 64 are rotated 180 degrees from concave air moving surface 138 of blades 136 of impeller 74 of blower 66. As a result, when electrical current having a first polarity is provided to motor 62 and motor 62 operates to drive rotation of drive shaft 70 in a clockwise direction, i.e., the direction of arrow A of Fig. 6, concave air moving surfaces 138 of blades 136 of impeller 72 act as the leading surfaces of blades 136 as blades 136 move through the air. As a result, impeller 72 and blower 64 are operating in a substantially efficient manner and air is drawn through inlet 118 in blower 64 and discharged through outlet 114 in blower 64.

Specifically, referring to Fig. 15, in order to draw air through blower 64, volute housing 110 begins at a point adjacent to cutoff 142, where inner surfaces 106, 124 of inner and outer housing portions 76, 80 are initially positioned substantially adjacent blades 136 of impeller 72. As impeller 72 is rotated clockwise, a radially increasing gap 144 is formed between the radial outer edge of blades 136 and inner walls 106, 124 of inner and outer housing portions 76, 80. This allows for an increased volume of air to be moved by impeller 72 as blades 136 of impeller 72 rotate. The air moved by impeller 72 is directed by housing portions 76, 78 in the direction of outlet 114 where it is discharged from blower 64. As the air is discharge from outlet 114 of blower 64, additional air is drawn into volute 110 formed by inner and outer housings 76, 80, allowing the flow of air through blower 64 to continue. Additionally, due to the small size of gap 144 adjacent to outlet 114, cutoff 142, which was discussed briefly above, is created such that any air moved by impeller 72 in the direction of outlet 114 will exit through outlet 114 and any air positioned clockwise of cutoff point 142 is further accelerated by impeller 72 in the direction of outlet 114.

In contrast to blower 64, with motor 62 operating to drive rotation of drive shaft 70 in a clockwise direction, i.e., the direction of arrow A of Fig. 6, convex surfaces 140 of blades 136 of impeller 74 define the leading surfaces of impeller 74 as blades 136 move through the air. However, because convex surfaces 140 of blades 136 are the first to contact the air within blower 66, little movement of air within blower 66 is generated. As a result, blower 66 is operating to move substantially no air through blower 66, i.e., from inlet 120 of outer housing portion 82 to outlet 116 of inner and outer housing portions 78, 82. Stated another way, while blower 66 is operating, i.e., impeller 74 is rotating, the efficiency of blower 66 is substantially zero and no appreciable amount of air is exiting outlet 116. In exemplary embodiments, the velocity of air discharged by blower 64, i.e., generated by impeller 72, when shaft 70 is rotated in a clockwise direction may be as much as two times, three times, four times, five times, six times, seven times, eight times, nine times, or ten times greater than the velocity of air discharged by blower 66, i.e., generated by impeller 74. In one exemplary embodiment, when shaft 70 is rotated in a clockwise direction, blower 64 and impeller 72 generate an air flow having a velocity at discharge of 3.0 meters per second and blower 66 and impeller 74 generate an air flow having a velocity at discharge of 0.5 meters per second, which results in blower 64 and impeller 72 generating air flow having a velocity that is six times greater than blower 66 and impeller 74. Therefore, in order for blower 66 to effectively operate to draw air therethrough and discharge the same at an effective velocity, the rotation of drive shaft 70 of motor 62 must be reversed.

In order to reverse the rotation of shaft 70 and motor 62, the polarity of the electric current provided to motor 62 is reversed. By reversing the polarity of electric current provided to motor 62, the rotation of shaft 70 of motor 62 is correspondingly reversed. Specifically, by reversing the polarity of the electric current provided by motor 62, drive shaft 70 will rotate in a counterclockwise direction, i.e., the direction of arrow B in Fig. 6. With shaft 70 of motor 62 rotating in a counterclockwise direction, impeller 74 of blower 66 is also rotating in a counterclockwise direction. As impeller 74 moves in a counterclockwise direction, concave air moving surfaces 138 of blades 136 of impeller 74 define the leading surfaces of impeller 74 and are advanced through the air within blower 66. As a result, air will be drawn from inlet 120 in outer blower housing 82, through blower 66, and discharged through outlet 116 defined by inner and outer housing portions 78, 82, in a substantially similar manner as described in detail above with respect to blower 64. In contrast, with impeller 72 of blower 64 rotating in a counterclockwise direction, i.e., the direction of arrow B of Fig. 6, convex surfaces 140 of blades 136 of impeller 72 define the leading surfaces of impeller 72 and are advanced through the air within housing 64. As a result, blower 64 is operating to move substantially no air through blower 64, i.e., from inlet 118 of outer housing portion 80 to outlet 114 defined by inner and outer housing portions 76, 80. Stated another way, while blower 64 is operating, i.e., impeller 72 is rotating, the efficiency of blower 64 is substantially zero and no appreciable amount of air is exiting outlet 114. In exemplary embodiments, the velocity of air discharged by blower 66, i.e., generated by impeller 74, when shaft 70 is rotated in a counterclockwise direction may be as much as two times, three times, four times, five times, six times, seven times, eight times, nine times, or ten times greater than the velocity of air discharged by blower 64, i.e., generated by impeller 72. In one exemplary embodiment, when shaft 70 is rotated in a counterclockwise direction, blower 66 and impeller 74 generate an air flow having a velocity at discharge of 3.0 meters per second and blower 64 and impeller 72 generate an air flow having a velocity at discharge of 0.5 meters per second, which results in blower 66 and impeller 74 generating air flow having a velocity that is six times greater than blower 64 and impeller 72.

Advantageously, dual centrifugal blower 60 allows for the selective discharge of air through outlets 114, 116. As described in detail below, dual centrifugal blower 60 may be used with air fresheners 150, 250, described below, to alternatingly disperse a fragrance or other volatile fluid into the ambient environment. For example, dual centrifugal blower 60 may be used to alternatingly disperse different first and second fragrances. By alternatingly dispensing a first and second fragrance, olfactory fatigue, i.e., the temporary normal inability to distinguish a particular odor after a prolonged exposure to that airborne compound may be prevented.

### TABLE-TOP AIR FRESHENER

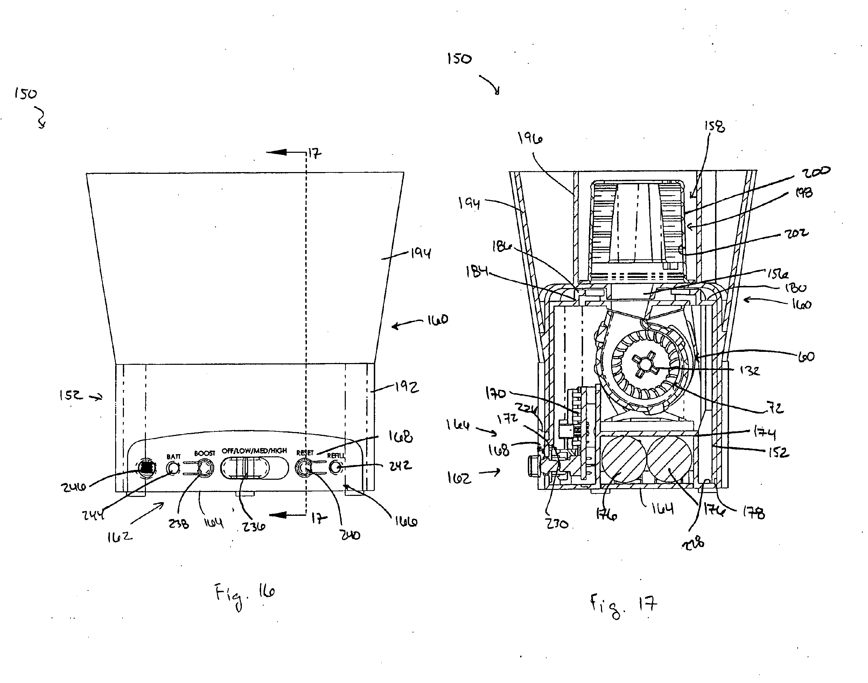

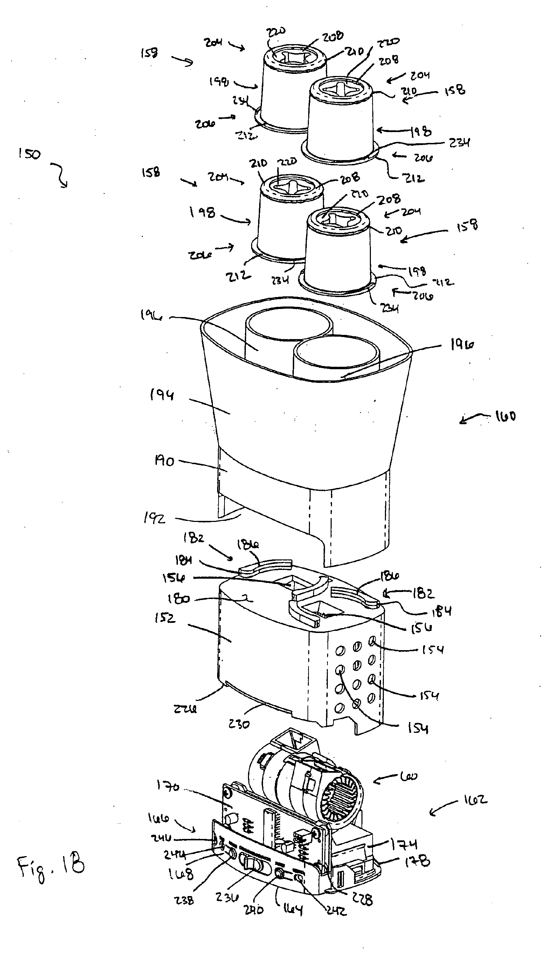

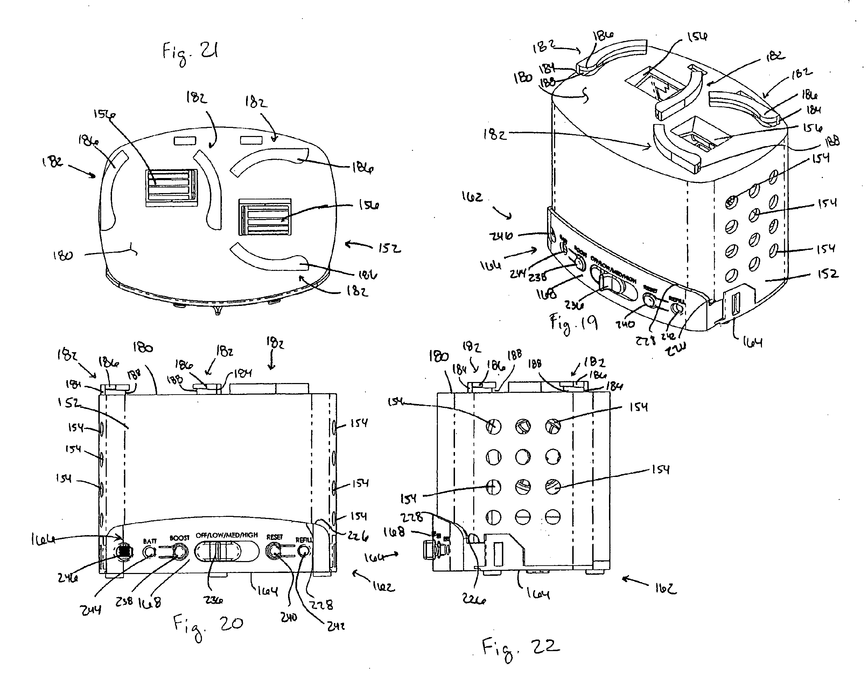

Referring to Figs. 16-27, a "residential" or table-top air freshener 150 is shown. While identified herein as a table-top air freshener, air freshener 150 is designed to be positioned on any flat surface, such as a desk-top, a dresser, a floor, or a bookshelf, and may be used to dispense any volatile fluid into the air. Referring to Fig. 18, air freshener 150 includes housing 152 having inlets 154 and outlets 156. During operation of air freshener 150, air is drawn into inlets 154 and is discharged out of outlets 156, where it passes through air freshener canisters 158 positioned within shroud 160 and is then discharged into the ambient environment.

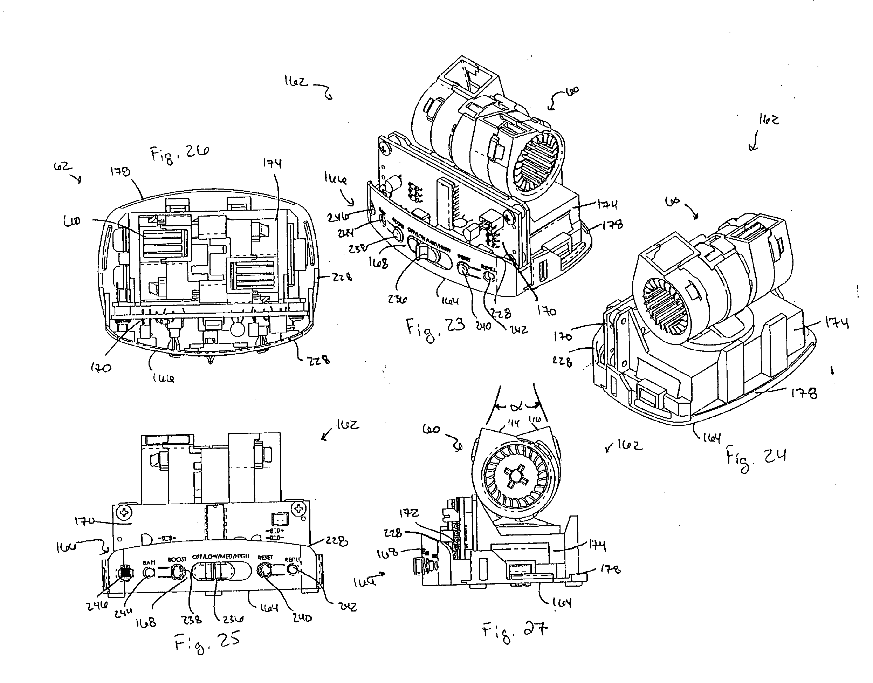

Referring to Figs. 23-27, air freshener 150 includes base 162 to which housing 152 is attached. Base 162 defines a substantially flat, level bottom surface 164 upon which the remainder of air freshener 150 is supported. Positioned on forward face 166 of air freshener 150 is control panel 168, the operation of which is described in detail below in conjunction with the operation of air freshener 150. Control panel 168 is connected to circuit board 170, and provides inputs to circuit board 170 that control the operation of air freshener 150. Control panel 168 also includes internal groove 172, which is designed to facilitate securement of housing 152 thereto, as described in detail below.

In one exemplary embodiment, base 162 also includes cover 174 that is removeable from base 162 and is designed to cover and hide a power supply, such as batteries 176 thereunder. As shown in Fig. 17, batteries 176 may be positioned under cover 178 and connected to circuit board 170 to provide direct current to circuit board 170 for controlling to operation of air freshener 150. In addition to cover 178, an access may be provided to batteries 176 from flat bottom surface 164 of base 162. For example, another removeable cover (not shown) may be positioned adjacent to batteries 176 and may define a portion of bottom surface 164 of base 162 when secured to base 162. Alternatively or in addition to batteries 176, an alternating current power supply (not shown) may be provided that receives alternating current from a known receptacle, such as a wall socket, and provides the same to circuit board 170. Base 162 also includes rearwardly extending arcuate flange 178, which is also configured to facilitate securement of base 162 to housing 152, as described in detail below.

Referring to Figs. 23-27, dual centrifugal blower 60 is secured to base 162 to provide airflow through air freshener 150. Dual centrifugal blower 60 may be secured to base 162 in any known manner, such as by forming a snap-fit or friction-fit with a portion of base 162, by fasteners, or by a bayonet connection, for example. In one exemplary embodiment, dual centrifugal blower 60 is positioned atop cover 174 of base 162 and is electrically connected to circuit board 170 by electrical leads 67 (Fig. 6). Circuit board 170 controls the operation of dual centrifugal blower 60 by providing electrical current to motor 62 of dual centrifugal blower 60 through electrical leads 67 and/or by reversing the polarity of the electrical current provided to dual centrifugal blower 60, as described in detail below with respect to the operation of air freshener 150. Additionally, as shown in Figs. 23, 24, and 27, dual centrifugal blower 60 is positioned within air freshener 150 with outlets 114, 116 rotated relative to one another such that angle α is formed. In one exemplary embodiment, angle α is approximately thirty degrees. In other exemplary embodiments, angle α may be any angle that can be formed between outlets 114, 116 in which outlets 114, 116 are still positioned to discharge air through outlets 156 of housing 152. By forming an angle α between outlets 114, 116, blowers 64, 66 of dual centrifugal blower 60 function to direct the airflow generated by blowers 64, 66 at the sidewalls of fragrance canisters 158 and entrain a greater amount of volatile fluid within air passing through fragrance canisters 158, as described in detail below.

Referring to Figs. 18-22 and as indicated above, housing 152 is shown and defines inlets 154 and outlets 156. Housing 152 is designed to be secured to base 162 and functions to direct airflow from inlets 154 to outlets 156. Additionally, housing 152 also acts as a cover for circuit board 170 and dual centrifugal blower 60, as well as the other components of base 162. Housing 152 includes upper surface 180 in which outlets 156 are formed. Engagement members, such as L-shaped flanges 182, extend upwardly from upper surface 180 of housing 152. In one exemplary embodiment, a pair of opposing L-shaped flanges 182 are positioned adjacent to each of outlets 156, with one of the pair of opposing L-shaped flanges 182 positioned adjacent to a first side of each of outlets 156 and the other of the pair of opposing L-shaped flanges positioned adjacent to a second, opposite side of each of outlets 156. Additionally, L-shaped flanges 182 have a generally arcuate shape and include upwardly extending base portions 184 and outwardly extending arm portions 186. Base portions 184 function to space arm portions 186 a predetermined distance from upper surface 180 of housing 152. As a result, slots or gaps 188 are formed between arms portions 186 of L-shaped flanges 182 and upper surface 180 of housing 152. L-shaped flanges 182 of housing 152 facilitate the positioning and retention of fragrance canisters 158 atop housing 152, as described in detail below.

Referring to Figs. 16-18, shroud 160 is shown, which is designed to be positioned over housing 152 and at least partially over base 162. Shroud 160 includes shroud body 190 having control panel cutout 192 formed therein. Surround 194 extends upwardly from shroud body 190 and defines a perimeter wall that diverges outwardly in a direction extending away from shroud body 190. Fragrance canister isolators 196 are positioned within surround 194 of shroud 160. Fragrance canister isolators 196 are formed as a pair of cylindrical members connected to and extending upwardly from shroud body 190. Fragrance canister isolators 196 are sized to receive fragrance canisters 158 therein and to allow L-shaped flanges 182 of housing 152 to pass therethrough and extend upwardly into fragrance canister isolators 196. Advantageously, fragrance canister isolators 196 act to shield fragrance canisters 158 from moving air that may otherwise contact fragrance canisters 158. As a result, the air within fragrance canisters 158 that has, over time, become saturated with volatile fluid is unlikely to be disturbed by passing air, increasing the useful life of fragrance canisters 158. Further, shroud 160 also provides an aesthetic benefit by hiding fragrance canisters 158, housing 152, and portions of base 162.

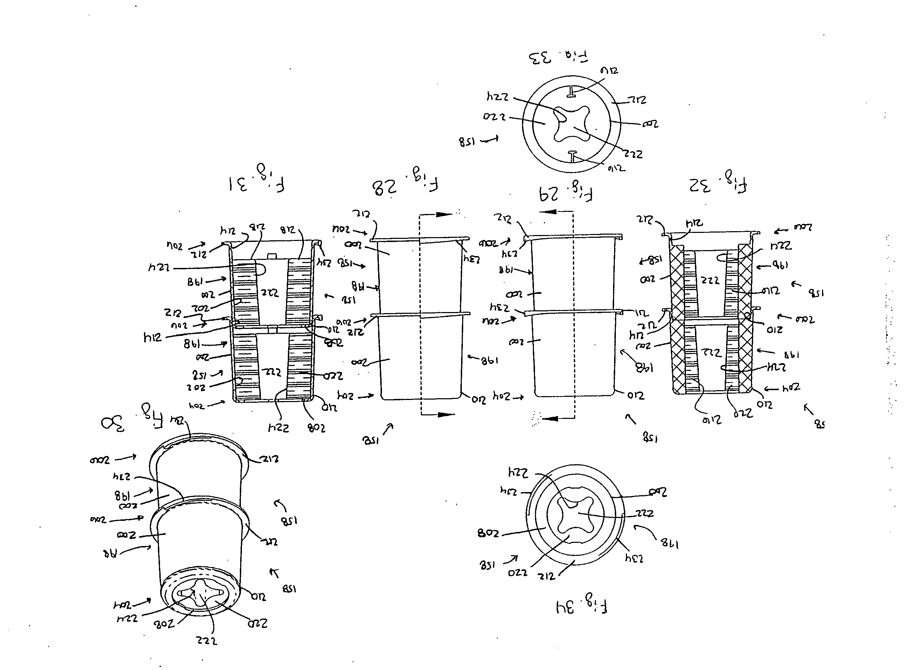

Referring to Figs. 28-34, fragrance canisters 158 are shown including canister bodies 198 having exterior walls 200, interior walls 202, upper ends 204, and lower ends 206. Canister bodies 198 are gradually tapered in a direction extending from lower ends 206 to upper ends 204. Upper ends 204 of fragrance canisters 158 include radially inwardly extending rims 208 and outwardly extending annular bulbous projections 210 defined by exterior walls 200. Lower ends 206 of fragrance canister 158 include outwardly extending radial rims 212 and interior annular grooves 214 defined by interior walls 202. Additionally, extending from opposing sides of interior walls 202 of canister bodies 198 are T-bars 216. T-bars 216 provide an increased surface area that facilitates the securement and retention of a fragrance gel or other volatile fluid within fragrance canisters 158. T-bars 216 extend from upper ends 204 of fragrance canisters 158 toward lower ends 206 thereof and terminate prior to reaching outwardly extending radial rims 212 to define shoulders 218, which, in conjunction with bulbous projections 210 and grooves 214, facilitate the stacking of fragrance canisters 158 atop one another, as described in detail below.

Positioned within bodies 198 of fragrance canisters 158 is fragrance gel 220. While described herein as containing fragrance gel 220, fragrance canister 158 may be used in conjunction with any volatile fluid that may be advantageously released into the ambient environment, such as a scented liquid, a disinfectant, an insecticide, or an insect repellant, for example. Fragrance gel 220 may be formed of a matrix of polymer material having a volatile fluid that is impregnated within the polymer material. In one exemplary embodiment, the volatile fluid may form as little as 10%, 15%, 20%, 25%, 30%, 35%, 40%, or 45% of the total weight of fragrance canisters 158 or may form as much as 95%, 90%, 85%, 80%, 75%, 70%, 65%, 60%, 55%, or 50% of the total weight of fragrance canisters 158, for example, with the remainder formed from the combination of the polymer material and canister bodies 198. In one embodiment, the greater the percentage of the total weight of fragrance canisters 158 that is formed by the volatile fluid, the larger the effective life of fragrance canisters 158. The volatile fluid may be, for example, a liquid fragrance commercially available as 190196K Sunshine Fruits fragrance from Firmenich Company of Geneva, Switzerland. The polymer material into which the volatile fluid is impregnated may be formed as a plastic commercially available as Pebax 2533SN01 Polyether Block Amide from Arkema Inc. of Philadelphia, Pennsylvania.

Fragrance gel 220 is positioned within fragrance canisters 158 and defines interior passage 222 extending therethrough. Fragrance gel 220 may be formed with undulated interior surface 224, such that the portion of fragrance gel 220 defining interior passage 222 has an undulated surface. By forming interior surface 224 of fragrance gel 220 to be undulated, the surface area of interior surface 224 is increased and the amount of volatile fluid that is readily available to be released into the air within interior passage 222 is correspondingly increased. In other exemplary embodiment, fragrance gel 220 may be porous or have other known configurations that increase the surface area of interior surface 224 of fragrance gel 220.

In order to assemble desk top air freshener 150, housing 152 is aligned with base 162 and advanced in the direction of base 162. Specifically, housing 152 is advanced until lower edge 226 of housing 152 contacts upper edge 228 of base 162. Once in this position, additional advancement of housing 152 causes projection 230 of housing 152 to be inwardly deformed. Housing 152 is advanced until projection 230 is received within groove 172 of control panel 168 of base 162 and flange 178 of base 162 is received within a recess formed within housing 152.

With housing 152 secured to base 162, fragrance canisters 158 are then positioned atop upper surface 180 of housing 152. In order to retain fragrance canisters 158 on housing 152, outwardly extending radial rims 212 of fragrance canisters 158 are configured for receipt within gaps 188 defined between upper surface 180 of housing 152 and arm portions 186 of L-shaped flanges 182. Once fragrances canisters 158 are positioned atop upper surface 180 of housing 152, fragrance canisters 158 are then slid along upper surface 180 of housing 152 such that lower outwardly extending radial rims 212 of fragrance canisters 158 are received within gaps 188 defined between upper surface 180 and arm portions 186 of L-shaped flanges 182. Fragrance canisters 158 are advanced until movement of fragrance canisters 158 is prevented by L-shaped flanges 182 and/or interior passage 222 of fragrance canisters 158 are aligned with outlets 156 of housing 152. By positioning radial rims 212 of fragrance canisters 158 within gaps 188 defined by L-shaped flanges 182, axial movement of fragrance canisters 158 in a direction away from upper surface 180 of housing 152 may be prevented by the interaction outwardly extending radial rims 212 with arm portions 186 of L-shaped flanges 182.

In one exemplary embodiment outwardly extending radial rims 212 of fragrance canisters 158 include upper surfaces 234. Referring to Figs. 28-30, a portion of upper surfaces 234 of radial rims 212 increases in thickness as radial rims 212 advance radially around lower ends 206 of fragrance canisters 158. As outwardly extending radial rims 212 increase in thickness, a ramped surface is defined by radial rims 212. In other exemplary embodiments of air freshener 150, fragrance canisters 158 may be positioned with outwardly extending radial rims 212 positioned within an opening, such as gap 188 defined by L-shaped flanges 182, and then fragrance canisters 158 may be rotated approximate 45 degrees to form a friction fit with the retention feature defining the opening, such as arm portions 186 of L-shaped flanges 182. In this manner, an additional connection mechanism is provided to connect fragrance canisters 158 to housing 152.

Additionally, in one exemplary embodiment, a plurality of fragrance canisters 158 may be positioned atop one another to form a stacked configuration atop housing 152. In order to secure fragrance canisters 158 to one another, a first fragrance canister 158 is positioned below a second fragrance canister 158 and the two fragrance canisters 158 are axially aligned with one another. The fragrance canisters 158 are then advanced toward one another such that bulbous projection 210 of the first fragrance canister 158 is positioned adjacent to interior wall 202 of the second fragrance canister 158 and advanced therealong. As the fragrance canisters 158 continue to advance toward one another, bulbous projection 210 of the first fragrance canister 158 is ultimately received within groove 214 of the second fragrance canister 158 and, correspondingly, radially inwardly extending rim 208 of the first fragrance canister 158 contacts shoulder 218 of the second fragrance canister 158. Once in this position, the first and second fragrance canisters 158 are releaseably connected to one another.

With a desired number of fragrance canisters 158 positioned atop housing 152, shroud 160 may be positioned over canisters 158, housing 152, and base 162. Additionally, different designs of shroud 160 may be provide that are design to accommodate different numbers of fragrance canisters 158. For example, in one exemplary embodiment, a first shroud 160 may be provided that is designed to receive a single layer of fragrance canisters within fragrance canisters isolators 196 and surround 194. Additionally, a second shroud 160 may be provided in which fragrance canister isolators 196 and surround 194 are extended to receive sets of stacked fragrance canisters 158. Once a shroud 160 having the desired design is selected, fragrance canister isolators 196 are aligned with the fragrance canisters 158 and shroud base 162 is aligned is with housing 152. Shroud 160 is then advanced in the direction of base 162. Specifically, shroud 160 is advanced until the surface defining control panel cutout 192 of shroud body 190 contacts upper surface 180 of control panel 168 of housing 152. Once in this position, shroud 160 effectively surrounds at least a portion of base 162, housing 152, and fragrance canisters 158 providing aesthetic improvements to desk top air freshener 150 and isolating fragrance canisters 158 from one another and from passing ambient air.

The operation of desk-top air freshener 150 will now be described in detail. Referring to control panel 168 and Fig. 16, control panel 168 includes off/low/med/high slide switch 236, boost button 238, reset button 240, refill indicator light 242, low battery indicator light 244, and photo cell 246. When slide switch 236 is actuated to one of the "low", "med", or "high" positions, electrical current from batteries 176 is supplied to circuit board 170, which controls the operation of dual centrifugal blower 10. As indicated above, in other exemplary embodiments, batteries 176 may be replaced by or used in conjunction with a power supply that is connected to an alternating current power source, such as through a known electrical receptacle. In one exemplary embodiment, circuit board 170 is configured to provide electrical current to motor 62 in a first direction for a predetermined dispersion time after the passage of a predetermined diffusion time.

Specifically, when dual centrifugal blower 60 is not operating, volatile fluid contained within fragrance gel 220 of air freshener canister 158 diffuses into interior passages 222 of air freshener canister 158. However, after the passage of the predetermined diffusion time, the volatile fluid has reached equilibrium with the air within interior passages 222. Thus, regardless of any further passage of time, substantially no additional volatile fluid will diffuse into the air. Once the diffusion time has elapsed, circuit board 170 may provide electrical current having a first polarity to motor 62 of dual centrifugal blower 60. As a result, shaft 70 of motor 62 is driven in a clockwise direction and blower 64 of dual centrifugal blower 60 draws air through inlets 154 of housing 152 and forces the same through outlet 156 in housing 152. Additionally, due to the endpoint of angle α formed between outlets 114, 116 of motor 62 being positioned above outlets 114, 116, air discharged by blower 64 is directed through outlet 156 along a path that causes the air to contact interior surface 224 of fragrance gel 220 positioned within fragrance canister 158. As the airflow generated by blower 64 forces the air within interior passage 222, which now contains the diffused volatile fluid, out of interior passage 222 and fragrance canister 158, the fresh air discharged by blower 64 into fragrance canister 158 entrains additional volatile fluid. This air is then discharged from fragrance canister 158 and into the ambient environment. Advantageously, as indicated above, the outlet flow of air from blower 64 is directed to contact interior surface 224 of fragrance gel 220, which allows the air to entrain a greater amount of volatile fluid than is otherwise possible by directing the outlet flow of air directly through the center of interior passage 222 of fragrance canister 158.

Additionally, as indicated above, with shaft 70 of motor 62 rotating in a clockwise direction, blower 66 does not generate sufficient airflow to disperse the volatile fluid into the ambient environment and any air present within interior passage 222 of fragrance canister 158 positioned above outlet 156 that is saturated with volatile fluid remains substantially entirely within interior passage 222. Once the dispersion time has elapsed, circuit board 170 may stop the flow of electrical current to motor 62.

In this manner, blower 64 may be actuated to disperse volatile fluid into the ambient environment for a predetermined blower time. Additionally, depending on whether slide switch 236 is moved to the "low", "med", or "high" setting, the dispersion time may be varied. For example, by selecting the "med" setting, a dispersion time is selected that is greater than the dispersion time for the "low" setting and less than the dispersion time for the "high" setting. Additionally, circuit board 170 may be configured to provide current to motor 62 having a first polarity at predetermined times over a 45 minute interval, for example. Thus, in one exemplary embodiment, once the predetermined diffusion time, such as five minutes, has passed, circuit board 170 may provide current to motor 62 having a first polarity to cause shaft 70 of motor 62 to rotate in a first, clockwise direction and cause blower 64 to generate sufficient airflow to disperse the volatile fluid into the ambient environment for a 30 second dispersion time. This cycle is then repeated until the predetermined blower time, e.g., a 45 minute time interval, has passed.

Once the predetermined blower time, e.g., 45 minutes, has passed, circuit board 170 may be programmed to reverse the polarity of current provided to motor 62, which correspondingly reverses the rotation of shaft 70 and causes shaft 70 to rotate in a second, counterclockwise direction. Thus, in this configuration, blower 66 will generate sufficient airflow to disperse a volatile fluid into the ambient environment in a substantially identical manner as blower 64 described in detail above. However, as indicated above, with shaft 70 rotating in a second, counterclockwise direction, blower 64 does not generate sufficient airflow to disperse a volatile fluid into the ambient environment. Once the dispersion time has elapsed, circuit board 170 may stop the flow of electrical current to motor 62. This process may then be repeated for a predetermined blower time, e.g., a 45 minute time interval, as described above. Additionally, this pattern of alternating the polarity of the current provided to motor 62 based on the passage of a predetermined blower time and, correspondingly, alternating the dispersal of a volatile fluid into the ambient environment between blowers 64, 66, may continue indefinitely.

Advantageously, by utilizing both of blowers 64, 66, different air freshener canisters 158 may be used. Thus, a first one of air freshener canisters 158 may provide a first scent and a second one of air freshener canisters 158 may provide a second scent. This allows for air freshener 150 to disperse different fragrances into the ambient environment. As a result, individuals will not experience olfactory fatigue, i.e., they will not become acclimated to a specific volatile fluid, e.g., an air freshener, and, as a result, will continue to notice the improvement in air fragrance and/or disinfection, for example. Additionally, in another exemplary embodiment, blowers 64, 66 may be operated in a back-to-back manner, such that the first and second scents are dispersed from air freshener 150 and allowed to mix together in the ambient environment.

As indicated above, control panel 168 also includes boost button 238. When boost button 238 is depressed, control panel 168 activates one of blowers 64, 66 and allows for the discharge of volatile fluid into the air in the manner set forth above for a predetermined boost time. In one exemplary embodiment, the boost time is substantially equal to the blower time. Circuit board 170 also monitors the current provided by batteries 176 to circuit board 170. When the current provided by batteries 176 drops below a predetermined level, circuit board 170 activates low battery indicator light 244, which signals to the user that the batteries in air freshener 150 need to be changed.

Additionally, circuit board 170 monitors the passage of time from the last time that reset button 240 has been depressed. Once a predetermined replacement time has passed, circuit board 170 activates refill indicator light 242, which signals to the user that fragrance canisters 158 need to be refilled and/or replaced. Once fragrance canisters 158 are refilled and/or replaced, the user may depress reset button 240 and circuit board 170 will reset the replacement time and deactivate refill indicator light 242. In addition to monitoring the passage of time since reset button 240 has been depressed for purposes of activating refill indicator light 242, circuit board 170 may also track the passage of time since reset button 240 has been depressed to alter the length of the blower time, i.e., the amount of time that blowers 64, 66 are active. Specifically, as the amount of time that has passed since reset button 240 has been pressed increases, circuit board 170 corresponding increases the blower time in order to compensate for the decreasing amount of volatile fluid contained within fragrance gel 220. This allows for air freshener 150 to discharge substantially similar amounts of volatile fluid from fragrance canisters 158 irrespective of the amount of time that fragrance canisters 158 have been in use.

Further, circuit board 170 may also monitor that level of surrounding ambient light via photo cell 246 to determine whether it is daytime or nighttime and/or whether any artificial lights have been turned on. If photo cell 246 detects a sufficient amount of ambient light, circuit board 170 operates air freshener 150 as indicated above. However, if a sufficient amount of ambient light is not detected by photo cell 246, circuit board 170 may increase the diffusion time, i.e., the amount of time that is allowed to pass between successive operations of dual centrifugal blower 60. This functions to prolong the life of air freshener 150, fragrance canisters 158, and batteries 176.

In addition to operating dual centrifugal blower 60 as described in detail above, circuit board 170 may be programmed to operate dual centrifugal blower 60 in any desired manner. For example, circuit board 170 may be configured to operate dual centrifugal blower 60 such that at least one of blowers 64, 66 is operating at all times. Alternatively, the time between operation of blowers 64, 66 may be increased or decreased. Additionally, the diffusion times, dispersion times, and blower times may be set to last for any desired length of time.

While the operation of air freshener 150 is described in detail herein with specific reference to dual centrifugal blower 60, dual centrifugal blower 10 may also be used in conjunction with air freshener 150 in a substantially similar manner as dual centrifugal blower 60 described in detail above.

### WALL MOUNTED AIR FRESHENER

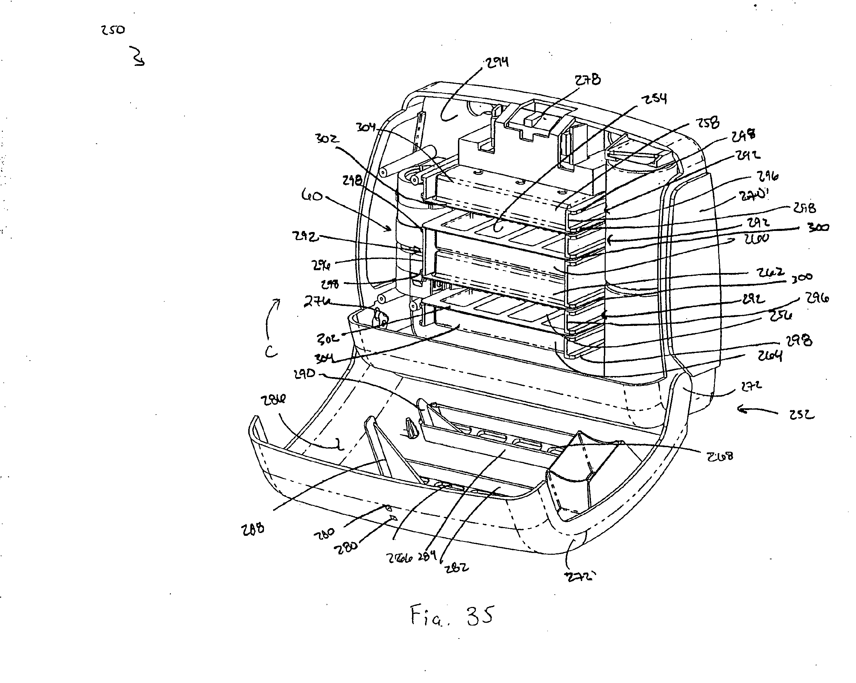

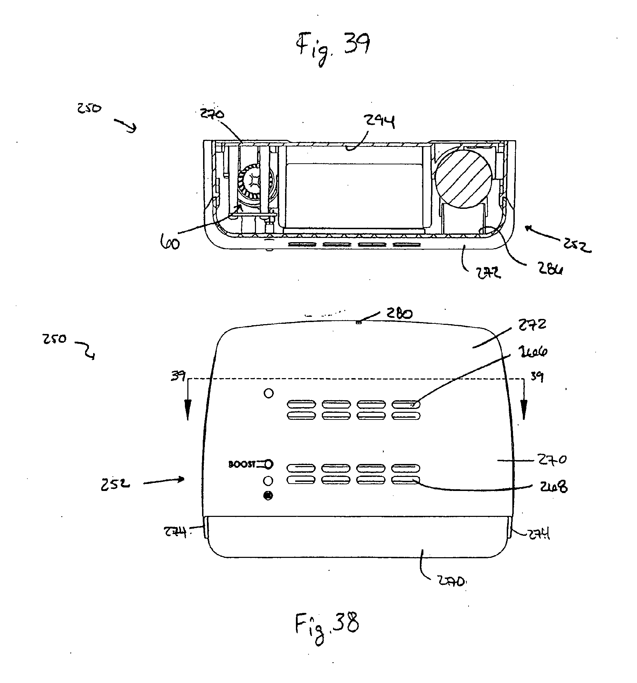

Referring to Fig. 35-39, a "commercial" or wall mounted air freshener 250 is shown, which incorporates dual centrifugal blower 60 of Figs. 1-15 to discharge a volatile fluid into the ambient environment. During operation of air freshener 250, air is drawn into clamshell housing 252, where it passes through air channels 254, 256 defined at least partially by fragrance cartridges 256, 258, 260, 262, and is then discharged into the ambient environment through outlets 266, 268. While described herein as being an air freshener, air freshener 250 may be used to distribute any other known volatile fluids that provide a beneficial effect, such as disinfectants, insecticides, or insect repellants, for example, into the ambient environment.

Referring to Fig. 35, air freshener 250 includes clamshell housing 252 having base 270 and lid 272. Base 270 is configured to be secured to a wall of building, such as a bathroom or kitchen wall, using known fasteners, such as screws, nails, or bolts, that extend through openings (not shown) in base 270. Lid 272 of housing 252 is rotatably secured to base 270. Specifically, in one exemplary embodiment, ears 274 of lid 272 are rotatable secured to base 270 at pivot points 276. Housing 252 may be opened and closed by rotating lid 272 about pivot points 276.

To retain lid 272 in a closed position, base 270 includes resiliently deformable lock portion 278. As lid 272 is advanced in the direction of arrow C of Fig. 35, lid 272 contacts resiliently deformable lock portion 278 of base 270 and, as lid 272 continues to advance, lock portion 278 is deformed. Once lid 272 has been sufficiently advanced to reach a closed position, resiliently deformable lock portion 278 is biased into a locking recess (not shown) formed in lid 272 and lid 272 is retained in the closed position. In order to place lid 272 in an open position and correspondingly open clamshell housing 252, openings 280 are provided in lid 272 and a key or other device may be passed through openings 280 to depress resiliently deformable lock portion 278 of base 270. With resiliently deformable lock portion 278 depressed inwardly in a direction away from openings 280, lid 272 may be rotated about pivot points 276 and placed in an open position.

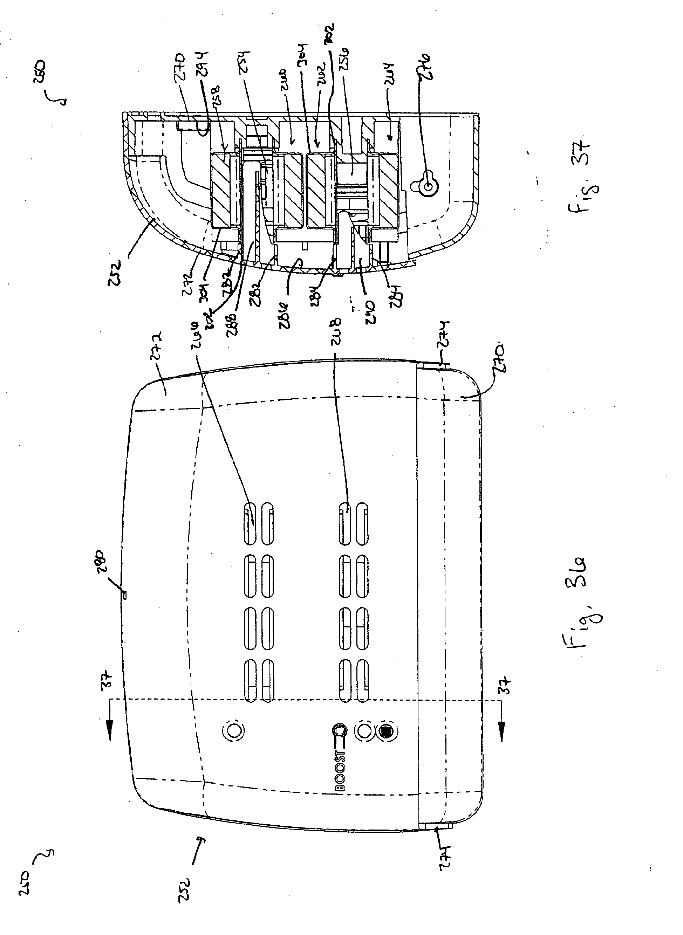

Referring to Figs. 35 and 37, flanges 282, 284 extend from inner surface 286 of lid 272 and surround outlets 266, 268, respectively, extending through lid 272. Outlets 266, 268 are designed to facilitate the release of a volatile fluid, such as air freshener, from housing 252, as described in detail below. In addition, lid 272 also includes projections 288, 290 that extend into the flowpath of air discharged by dual centrifugal blower 60 to increase the velocity of the air discharged therefrom, as described below. Referring to Fig. 36, lid 272 also includes control panel 273 positioned thereon.

Returning to base 270 of clamshell housing 252 and Fig. 35, base 270 includes a plurality of supports 292 extending inwardly in the direction of lid 272 from rear wall 294 of base 270. In one exemplary embodiment, supports 292 are formed as C-channel shaped projections. In this embodiment, each of supports 292 has a base body 296 and a pair of outwardly extending arms 298. Outwardly extending arms 298 of adjacent supports 292 cooperate to define gaps 300 therebetween. Gaps 300 are sized for receipt of rims 302 of air freshener cartridges 258, 260, 262, 264, as described in detail below..

Referring to Fig. 35, air freshener cartridges 258, 260, 262, 264 include outer shells 304 having perimeter rims 306 that extend outwardly therefrom. Fragrance gel 220, described in detail above with respect to table top air freshener 150 is contained within outer shells 304 of air freshener cartridges 258, 260, 262, 264. In one exemplary embodiment, outer shells 304 are formed from a polymer material and fragrance gel 220 is formed of a polymer material having a volatile fluid impregnated within the polymer material. In one exemplary embodiment, the polymer material that forms outer shells 304 is a polypropylene film. Additionally, the volatile fluid contained within fragrance gel 220 may form as little as 10%, 15%, 20%, 25%, 30%, 35%, 40%, or 45% of the total weight of air freshener cartridges 258, 260, 262, 264 or may form as much as 95%, 90%, 85%, 80%, 75%, 70%, 65%, 60%, 55%, or 50% of the total weight of air freshener cartridges 64, 66, 68, 70, for example, with the remainder formed from the combination of the polymer material of fragrance gel 220 and outer shells 304. In one embodiment, the greater the percentage of the total weight of air freshener cartridges 258, 260, 262, 264 that is formed by the volatile fluid, the larger the effective life of air freshener cartridges 258, 260, 262, 264.

The volatile fluid used to form fragrance gel 220 may be, for example, a liquid fragrance commercially available as 190196K Sunshine Fruits fragrance from Firmenich Company of Geneva, Switzerland. The polymer material into which the volatile fluid is impregnated to form fragrance gel 220 may be formed as a plastic commercially available as Pebax 2533SN01 Polyether Block Amide from Arkema Inc. of Philadelphia, Pennsylvania. Additionally, fragrance gel 220 of air freshener cartridges 258, 260, 262, 264 may be formed by heating and mixing the polymer material and the volatile fluid at approximately 300 °F. The mixture is then cooled to a gel or solid viscous form which can be formed into a desired shape, such as a tube. The desired shape can be further formed via a number of manufacturing methods, such as by compression molding, for example.

As shown in Fig. 35, dual centrifugal blower 60 is positioned within base 270 of clamshell housing 252 and adjacent to air freshener cartridges 258, 260, 262, 264. Dual centrifugal blower 60 may be secured to base 270 of wall-mounted air freshener dispenser 250 in any manner, such as by a snap fit, a friction fit, or the use of known fasteners. Specifically, referring to Fig. 5, dual centrifugal blower 60 is positioned with outlets 114, 116 of blowers 64, 66 aligned substantially parallel with one another, i.e., no angle α is defined between outlets 114, 116. Additionally, dual centrifugal blower 60 is positioned within base 270 of housing 252 such that outlets 114, 116 are aligned with and positioned at least partially between air freshener cartridges 258, 260 and 262, 264, respectively.

Specifically, air freshener cartridges 258, 260 are positioned adjacent outlet 114 and rotated 180° relative to one another. Similarly, air freshener cartridges 262, 264 are positioned adjacent outlet 116 and rotated 180° relative to one another. Air freshener cartridges 258, 260 cooperate with one of supports 292 extending from base 270, rear wall 294 of base 270, and flange 282 extending from lid 272 to define air channel 254 and air freshener cartridges 262, 264 cooperate with one of supports 292 extending from base 270, rear wall 294 of base 270, and flange 282 extending from lid 272 to define air channel 256. Referring to flanges 282, 284, with lid 272 in a closed position, as described in detail above, flanges 282, 284 contact rims 302 of air freshener cartridges 258, 260, 262, 264 and supports 292 to facilitate the formation of air channels 254, 256. Additionally, projections 288, 290 that extend from lid 272 may also extend into air channels 254, 256. In one exemplary embodiment, projections 288, 290 extend substantially adjacent to outlets 114, 116 of dual centrifugal blower 60 and restrict the passage of air out of outlets 114, 116. As a result, projections 288, 290 function to increase the speed of the air passing through air channels 254, 256.

Similar to air freshener 150, air freshener 250 may be powered by batteries 176 that are electrically connected to a circuit board (not shown). The circuit board of air freshener 250 may be substantially identical to circuit board 170 described in detail above with respect to air freshener 150 and has been omitted for clarity. Motor 62 of dual centrifugal blower 60 is electrically connected to the circuit board, such as through electrical leads 67 (Fig. 6), and receives electrical power through circuit board 170.

The operation of air freshener 250 will now be described in detail. Air freshener 250 is configured to be used in commercial applications, such as a bathroom of a restaurant or other commercial establishment, for example, and, therefore, the features of air freshener 250 may be more limited than the features of air freshener 150. However, air freshener 250 may be modified to operate in a substantially identical manner as air freshener 150.

In order to start the operation of air freshener 250, clamshell housing 252 is opened and a power switch (not shown) that is positioned within clamshell housing 252 is actuated to an "on" position. Clamshell housing 252 may then be closed. With the power switch actuated to an "on" position, electrical current from batteries 176 is supplied to circuit board 170, which controls the operation of dual centrifugal blower 60. As indicated above, in other exemplary embodiments, batteries 176 may be replaced by a power source that is connected to a source of alternating current, such as a known electrical receptacle. In one exemplary embodiment, circuit board 170 is configured to provide current having a first polarity to motor 62 to cause motor 62 to rotate shaft 70 in a first, clockwise direction for a predetermined dispersion time after the passage of a predetermined diffusion time.

Specifically, when dual centrifugal blower 60 is not operating, volatile fluid contained within air freshener cartridges 258, 260, 262, 264 diffuses into air channels 254, 256. However, after the passage of the predetermined diffusion time, the volatile fluid has reached equilibrium with the air within air channels 254, 256. Thus, regardless of any further passage of time, substantially no additional volatile fluid will diffuse into the air.

Thus, once the diffusion time has elapsed, circuit board 170 may provide a current having a first polarity to motor 62 to cause rotation of shaft 70 in a first, clockwise direction, as indicated above. As a result of shaft 70 rotating in a clockwise direction, blower 64 generates a sufficient amount of airflow to disperse volatile fluid into the ambient environment. Specifically, the airflow generated by blower 64 exits outlet 114 of blower 64 and enters air channel 254 where it forces the air within air channel 254, which now contains the diffused volatile fluid, out of outlet 266 in lid 272. However, as indicated above, with shaft 70 rotating in a clockwise direction, blower 66 does not generate sufficient airflow to disperse a volatile fluid into the ambient environment and any air present within air channel 256 that is saturated with volatile fluid remains substantially entirely within air channel 256. Once the dispersion time has elapsed, circuit board 170 may stop the flow of electrical current to motor 62.

In this manner, blower 64 may be actuated to disperse volatile fluid into the ambient environment for a predetermined blower time. Specifically, circuit board 170 may be configured to provide current to motor 62 in a first, clockwise direction at predetermined times over a 45 minute interval, for example. Thus, in this embodiment, once a diffusion time of five minutes, for example, has passed, circuit board 170 may provide current having a first polarity to motor 62 and blower 62 may then generate sufficient airflow to disperse the volatile fluid into the ambient environment for a 30 second dispersion time. This cycle is then repeated until the predetermined blower time, e.g., a 45 minute time interval, has passed.

Once the predetermined blower time has passed, circuit board 170 may be programmed to reverse the polarity of the current provided to motor 62, which correspondingly reverses the rotation of shaft 70 and causes shaft 70 to rotate in a second, counterclockwise direction. Thus, in this configuration, blower 66 generates a sufficient amount of airflow to disperse volatile fluid into the ambient environment, as described in detail above. Specifically, the airflow generated by blower 66 exits outlet 268 of blower 66 and enters air channel 256 where it forces the air within air channel 256, which now contains the diffused volatile fluid, out of outlet 268 in lid 272. However, as indicated above, with shaft 70 rotating in a second, counterclockwise direction, blower 64 does not generate sufficient airflow to disperse a volatile fluid into the ambient environment and any air present within air channel 254 that is saturated with volatile fluid remains substantially entirely within air channel 254. Once the dispersion time has elapsed, circuit board 170 may stop the flow of electrical current to motor 62.

This process may then be repeated for a predetermined blower time, e.g., a 45 minute time interval, as described above. Additionally, this pattern of alternating the polarity of the current provided to motor 62 based on the passage of a predetermined blower time and, correspondingly, alternating between blowers 64, 66 dispersing a volatile fluid into the ambient environment, many continue indefinitely.

Advantageously, by utilizing both of blowers 64, 66 different sets of air freshener cartridges 258, 260, and 262, 264, respectively, may be used. Thus, air freshener cartridges 258, 260 may provide a first scent and air freshener cartridges 262, 264 may provide a second scent. This allows for air freshener 250 to disperse different fragrances into the ambient environment. As a result, individuals will not experience olfactory fatigue, i.e., will not become acclimated to a specific volatile fluid, e.g., an air freshener, and, as a result, will continue to notice the improvement in air fragrance and/or disinfection, for example. Additionally, in another exemplary embodiment, blowers 64, 66 may be operated in a back-to-back manner, such that the first and second scents are dispersed from air freshener 250 and allowed to mix together in the ambient environment.

In addition to operating blower 250 as described in detail above, circuit board 170 may be programmed to operate blower 250 in any desired manner. For example, as shown in Fig. 36, lid 272 includes boost button 238 and photo cell 246 with function to alter the operation of air freshener 250 is a substantially identical manner as air freshener 150 described in detail above. Further, circuit board 170 may be configured to operate dual centrifugal blower 60 such that at least one of blowers 64, 66 is operating at all times. Alternatively, the time between operation of blowers 64, 66 may be increased or decreased. Additionally, the diffusion times, dispersion times, and blower times may be set to last for any desired length of time.

Additionally, while the operation of air freshener 250 is described in detail herein with specific reference to dual centrifugal blower 60, air freshener 250 may also be utilized in conjunction with dual centrifugal blower 10 as described in detail above.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A dual centrifugal blower (10, 60), including:
a motor (42, 62) having a housing and a shaft (40, 70), said motor operable to drive rotation of said shaft in a first direction (A);
a first impeller (36, 72) secured to said shaft in a first position, said first impeller having a first plurality of non-planar blades (48, 136) and disposed in a first housing (32, 110) having a first outlet (18, 114); and
a second impeller (38, 74) secured to said shaft in a second position, said second impeller having a second plurality of non-planar blades (50, 136) and disposed in a second housing (34, 112) having a second outlet (22, 116), **characterized by**:
said motor is operable to alternatingly drive rotation of said shaft in an opposite, second direction (B) and said second position of said second impeller is diametrically opposed to said first position of said first impeller, wherein, when said motor drives rotation of said shaft in said first direction, said first impeller is operable to generate an air flow having a greater velocity at said first outlet than said second impeller generates at said second outlet, and when said motor drives rotation of said shaft in said second direction, said second impeller is operable to generate an air flow having a greater velocity at said second outlet than said first impeller generates at said first outlet.

2. The dual centrifugal blower of Claim 1, **characterized in that** said second impeller is substantially identical to said first impeller.

3. The dual centrifugal blower of Claim 1, **characterized in that** said first plurality of non-planar blades comprise a plurality of arcuate blades each having a concave air moving surface (138) and an opposing convex surface (140), said concave air moving surfaces defining leading surfaces when said first impeller is rotated in said first direction and said convex surfaces defining leading surfaces when said first impeller is rotated in said second direction; and
said second plurality of non-planar blades comprise a plurality of arcuate blades each having a concave air moving surface (138) and an opposing convex surface (140), said concave air moving surfaces defining leading surfaces when said second impeller is rotated in said second direction and said convex surfaces defining leading surfaces when said second impeller is rotated in said first direction.

4. The dual centrifugal blower of Claim 1, **characterized in that**, when said motor drives rotation of said shaft in said first direction, said first impeller is operable to generate an air flow having a velocity at said first outlet that is at least twice as great as a velocity of the airflow at said second outlet generated by said second impeller, and when said motor drives rotation of said shaft in said second direction, said second impeller is operable to generate an air flow having a velocity at said second outlet that is at least twice as great as the velocity of the airflow at said first outlet generated by said first impeller.

5. The dual centrifugal blower of Claim 1, **characterized by** said shaft further comprising first and second opposite ends disposed externally of opposite sides of a housing of said motor, said first impeller secured to said first end of said shaft and said second impeller secured to said second end of said shaft.

6. The dual centrifugal blower of Claim 1, **characterized by** said first housing further comprising a first inlet (16, 118) and said second housing further comprising a second inlet (20, 120), wherein said first housing and second housing are substantially identical.

7. The dual centrifugal blower of Claim 1, **characterized by** said first impeller further comprising a first hub (132) and said second impeller further comprising a second hub (132), said first and second hubs positioned proximate said motor with said first plurality of non-planar blades and said second plurality of non-planar blades extending from said first and second hubs in respective directions away from said motor.

8. The dual centrifugal blower of Claim 1, **characterized in that** said first housing is positioned between said motor and said second housing, said first housing mounted to said motor.

9. The dual centrifugal blower of Claim 1, **characterized in that** said motor is positioned between said first housing and said second housing, each of said first and second housings mounted to said motor.

10. The dual centrifugal blower of Claim 9, **characterized in that** at least one of said first and second housings is rotatable relative to said motor, wherein rotation of said at least one of said first and second housings relative to said motor changes a relative positioning between said first and second outlets.

11. The dual centrifugal blower of Claim 9, **characterized by** each of said first and second housings further comprising a plurality of resiliently deformable projections (84) extending outwardly therefrom, said projections dimensioned to engage said motor, wherein said projections form a friction fit with said motor and connect said first and second housings to said motor.

12. The dual centrifugal blower of Claim 11, **characterized by** each of said first and second housings further comprising inner housing portions (76, 78) and outer housing portions (80, 82), said pluralities of resiliently deformable projections extending from said inner housing portions.

13. The dual centrifugal blower of Claim 12, **characterized in that** said inner housing portions and said outer housing portions of each of said first and second housings cooperate to define said first and second outlets.

14. The dual centrifugal blower of Claim 12, **characterized by** said inner housing portions further comprising at least one resiliently deformable tab (96) and said outer housing portions further comprising at least one opening (122), said at least one resiliently deformable projection of said inner housing portions configured for receipt within said at least one opening in said outer housing portions.

15. The dual centrifugal blower of Claim 9, **characterized in that** said first and second housings define volute housings, each of said first and second housings having an interior wall (106, 124), said interior wall of said first housing and said first plurality of non-planar blades cooperating to define a first gap (144) therebetween, said interior wall of said second housing and said second plurality of non-planar blades cooperating to define a second gap (144) therebetween, wherein said first gap increases circumferentially in said first direction from a first cutoff (142) defined by said first housing to said outlet of said first housing and said second gap increases circumferentially in said second direction from a second cutoff (142) defined by said second housing to said outlet of said second housing.
